# FASCICULE DE BREVET EUROPEEN

(11) **EP 3 688 141 B1**
(45) Date de publication et mention de la délivrance du brevet: **09.11.2022**
(21) Numéro de dépôt: 18780025.5
(22) Date de dépôt: 25.09.2018
(51) Int. Cl.: C12N 5/071, G01N 33/50

(54) **MODELE EX VIVO DE PEAU HUMAINE INFLAMMEE ET SES UTILISATIONS POUR LE CRIBLAGE DE COMPOSES ANTI-INFLAMMATOIRES**
EX-VIVO-MODELL EINER ENTZÜNDETEN MENSCHLICHEN HAUT UND DEREN VERWENDUNG ZUM SCREENING VON ENTZÜNDUNGSHEMMENDEN VERBINDUNGEN
EX-VIVO MODEL OF INFLAMED HUMAN SKIN AND USES THEREOF FOR SCREENING ANTI-INFLAMMATORY COMPOUNDS

(30) Priorité: 26.09.2017 FR 1771020
(43) Date de publication de la demande: 05.08.2020
(73) Titulaire: Genoskin, 31000 Toulouse (FR)
(72) Inventeur: JARDET, Claire, 31270 Cugnaux (FR); DESCARGUES, Pascal, Swampscott MA 01907 (US); NORSGAARD, Hanne, 2150 Nordhavn (DK); LOVATO, Paola, 2100 København (DK)
(74) Mandataire: Barbot, Willy
(86) Numéro de dépôt international: PCT/EP2018/000448
(87) Numéro de publication internationale: WO 2019/063122

(56) Documents cités:
- WO-A1-2014/182655
- ELLEN H. VAN DEN BOGAARD ET AL: "Crosstalk between Keratinocytes and T Cells in a 3D Microenvironment: A Model to Study Inflammatory Skin Diseases", THE JOURNAL OF INVESTIGATIVE DERMATOLOGY : OFFICIAL JOURNAL OF THE SOCIETY FOR INVESTIGATIVE DERMATOLOGY AND THE EUROPEAN SOCIETY FOR DERMATOLOGICAL RESEARCH, vol. 134, no. 3, 1 mars 2014 (2014-03-01), pages 719-727, XP055436202, US ISSN: 0022-202X, DOI: 10.1038/jid.2013.417 cité dans la demande
- KOENEN HANS J P M ET AL: "Human CD25highFoxp3pos regulatory T cells differentiate into IL-17-producing cells", BLOOD, 56TH ANNUAL MEETING OF THE AMERICAN-SOCIETY-OF-HEMATOLOGY; SAN FRANCISCO, CA, USA; DECEMBER 06 -09, 2014, vol. 112, no. 6, 15 septembre 2008 (2008-09-15), pages 2340-2352, XP002639440, ISSN: 0006-4971, DOI: 101.1182/BLOOD-2008-01-133967 [extrait le 2008-07-10]
- ELINE DESMET ET AL: "In vitro psoriasis models with focus on reconstructed skin models as promising tools in psoriasis research", EXPERIMENTAL BIOLOGY AND MEDICINE, vol. 242, no. 11, 1 juin 2017 (2017-06-01) , pages 1158-1169, XP055436191, GB ISSN: 1535-3702, DOI: 10.1177/1535370217710637
- TJABRINGA GEURANNE ET AL: "Development and validation of human psoriatic skin equivalents", AMERICAN JOURNAL OF PATHOLOGY; [10640], ELSEVIER INC, US, vol. 173, no. 3, 1 septembre 2008 (2008-09-01), pages 815-823, XP002539858, ISSN: 0002-9440, DOI: 10.2353/AJPATH.2008.080173
- LESLIE CALAPRE ET AL: "Heat-mediated reduction of apoptosis in UVB-damaged keratinocytes in vitro and in human skin ex vivo", BMC DERMATOLOGY, vol. 16, no. 1, 26 mai 2016 (2016-05-26), XP055436199, DOI: 10.1186/s12895-016-0043-4

## Description

Cette demande de brevet revendique la priorité de la demande de brevet français n°17/71020 déposée le 26 septembre 2017.

### DOMAINE DE L'INVENTION

La présente invention concerne un procédé d'obtention d'un modèle *ex vivo* de peau inflammée, un kit de mise en œuvre de ce procédé, ainsi que les utilisations du modèle de peau inflammée obtenu par le procédé de l'invention pour l'identification de composés présentant un effet anti-inflammatoire, en particulier pour le traitement du psoriasis.

### ART ANTERIEUR

Le psoriasis est une pathologie auto-immune inflammatoire chronique, touchant environ 2 % de la population européenne, caractérisée par une différenciation et une prolifération anormales des kératinocytes épidermiques se traduisant par la formation de plaques rouges souvent irritantes et par une accumulation de peaux mortes (les squames), lesquelles peuvent se situer sur tout le corps (le plus souvent sur les bras, le torse, les genoux, les pieds, les ongles, le visage et le cuir chevelu).

L'analyse histologique de ces plaques psoriasiques montre une augmentation d'épaisseur de l'épiderme, une différenciation incomplète des kératinocytes et un aspect festonné de la jonction dermo-épidermique, l'accumulation de polynucléaires neutrophiles dans l'épiderme et l'infiltration du derme par des cellules mononucléées, en particulier des macrophages et des lymphocytes T (LT), ainsi qu'une angiogenèse importante.

Les études immunohistochimiques confirment que le derme est le siège d'un infiltrat composé majoritairement de lymphocytes T CD4⁺ mémoires, de macrophages et de cellules dendritiques (DC) alors que les lymphocytes T CD8⁺ prédominent dans l'épiderme normal (non-inflammé).

L'inflammation du psoriasis est le résultat de l'interaction entre les cellules épithéliales (kératinocytes), les cellules dendritiques et les lymphocytes T. Au niveau moléculaire, ces interactions sont médiées par un réseau complexe de cytokines qui initient la pathologie et entretiennent un cercle vicieux d'activation cellulaire réciproque, pérennisant ainsi les lésions cutanées et leur chronicité (JF NICOLAS, Bull. Acad. Natle. Méd., 2014, 198 (1), p. 17-30). Les principales cytokines produites dans la peau psoriasique par les cellules dendritiques inflammatoires sont l'IL-12 et l'IL-23 qui jouent un rôle important dans la différenciation ou l'expansion des lymphocytes T vers les lignages Th1 (LTh1) ou Th17 (LTh2) (PECK and MELLINS, Immunology, 2009, 129, p. 147-153). Les LTh1 produisent de l'IFNγ, du TNFα et de l'IL-2 alors que les LTh17 sont responsables de la synthèse d'IL-17A, d'IL-17F, d'IL-21, d'IL-22 et dans une moindre mesure de TNFα. Cette production accrue de cytokines est à l'origine de l'activation des kératinocytes qui vont, en retour, donner lieu à une libération de peptides anti-microbiens, de médiateurs pro-inflammatoires (IL-1β, IL-6, IL-17C, TNFα, CXCL8, CXCL9, CXCL10, CXCL11 et CCL20) et de psoriasine (S100A7). Des études conduites en 2014 ont démontré que les cellules T CD4⁺ produisant l'IL-22 et les cellules CD8⁺ produisant l'IL-17 demeurent présentes dans la peau psoriasique précédemment lésionnelle et traitée par des thérapies psoriasiques efficaces. Ces résultats sont à rapprocher de ceux montrant que des greffes de peau non lésionnelle, d'apparence normale, provenant de patients atteints de psoriasis conduisent au développement de lésions psoriasiques chez des souris immunodéficientes. L'ensemble des ces travaux démontre que des cellules T résidentes de la peau persistent dans les lésions psoriasiques cliniquement résolues et produisent des cytokines connues pour causer la pathologie primaire de la maladie (Rachael A. CLARCK, Sci Transl Med., 2015, 7(269):269rv1).

Le traitement du psoriasis repose principalement sur l'utilisation de corticoïdes, d'inhibiteurs de phosphodiestérases, d'immunosuppresseurs (ciclosporine, tacrolimus) ou encore de photothérapie-UV. Plus récemment, des anticorps dirigés contre le TNFα (infliximab, adalimumab, étanercept, golimumab), l'IL-17-A (sécukinumab, ixekizumab), IL-12 / IL-23 (ustekinumab, guselkumab, tildrakizumab) ont été développés pour lutter contre les formes modérées à sévères du psoriasis ou sont en cours d'étude cliniques (GOODERHAM et al., Skin Therapy Letter, 2015, 20(1), p. 1-5). Certains de ces anticorps sont administrés par voie sous-cutanée pendant plusieurs semaines voire plusieurs années en fonction de la sévérité du psoriasis. Bien qu'ayant démontré une efficacité certaine, les traitements présentent des effets secondaires non négligeables et demeurent incapables de guérir du psoriasis. Il existe un réel besoin d'identifier de nouveaux composés susceptibles de palier à ces inconvénients. Pour ce faire, il est indispensable de disposer de modèle de peau inflammée, en particulier psoriasique.

Les modèles murins de psoriasis reposent sur des mutations spontanées (Scd1^{ab}/Scd1^{ab}, Sharpin^{cpdm}/Sharpin^{cpdm}, Ttc7^{fsn}/Ttc7^{fsn}) ou une modulation artificielle de l'expression de gènes impliqués dans différentes voies de signalisation, ou codant pour des cytokines ou des facteurs de croissance (MP SCHON, Experimental Dermatology, 2008, 17, p. 703-712). Toutefois, ces modèles ne présentent pas toutes les caractéristiques phénotypiques et histologiques du psoriasis rencontré chez l'homme ou bien présentent des caractéristiques supplémentaires limitant l'utilisation de ces modèles animaux pour le criblage de nouveaux composés. De plus, les différences physiologiques existant entre l'homme et l'animal ne permettent pas de prédire avec certitude la réponse immune chez l'homme, conduisant à l'abandon de nombre de composés candidats dès la phase préclinique de test.

Pour contourner ces difficultés, des xénotransplantations de peau humaine psoriasique ont été réalisées chez des souris nude ou SCID et ont permis de créer des modèles des formes aigues de psoriasis. Toutefois, le système immunitaire des souris receveuses étant incomplet, il est délicat là encore d'extrapoler les résultats obtenus à partir de ces modèles animaux à l'homme.

En outre, la prise en compte du bien-être animal associée à de nouvelles exigences réglementaires pousse au développement de modèles plus proche de la physiologie humaine. Ainsi, des modèles *in vitro* de peau artificielle ou encore d'épiderme ont été élaborés à partir de culture de kératinocytes et/ou de fibroblastes sur des matrices inertes de polycarbonate (SkinEthic^{™} RHE, EpiSkin^{™}, EPISKIN, France). Toutefois, de tels modèles ne contiennent pas les annexes épidermiques, ni les composantes du système immunitaire, limitant ainsi leur utilisation au criblage de composés potentiellement irritants.

Pour pallier ces inconvénients, les premiers modèles *ex-vivo* de peau humaine ont été réalisés à partir d'échantillons de peau humaine, par prélèvement du derme et de l'épiderme. Toutefois, la durée de vie de ces modèles n'excède pas 7 jours. Cette durée de vie a pu être augmentée en prélevant, en sus du derme et de l'épiderme, les annexes épidermiques et les follicules pileux (EP 2 019 316 B1). Toutefois, ces modèles ne correspondent pas à un état inflammatoire de la peau.

Des équivalents de peau humaine psoriasiques ont été obtenus *in vitro* soit par stimulation de fibroblastes dermiques sains, soit par utilisation de fibroblastes issus de patients atteints de psoriasis (TJABRINGA et al., Am. J. Pathol., 2008, 173, p. 815-823). Bien que ces modèles 3D montrent un épaississement de l'épiderme et une production de cytokines inflammatoires (TNFα, II-1α, IL-6, IL-8 et IL-22) caractéristiques de la pathologie, la composante immunitaire en reste absente.

Plus récemment, l'addition d'IL-1β directement dans le milieu de culture pendant plusieurs jours a permis d'induire une inflammation aigüe dans des explants de peau humaine (EP 2 885 637 B1). Maintenant, ce modèle ne permet pas de rendre compte de l'inflammation chronique observée dans le cas du psoriasis.

L'ajout de cellules T issues du sang périphérique, et activées ou différenciées en sous-populations Th1 ou Th17, sous le derme des modèles développés par Tjabringa *et al*. a permis de mettre en évidence l'influence exercée par les cytokines produites par ces cellules T sur les kératinocytes et la mise en place de l'inflammation (VAN DEN BOGAARD et al., J. Invest. Dermatol., 2014, 134, p. 719-727). Toutefois, certaines caractéristiques du psoriasis comme l'expression du marquer Ki67, l'hyperprolifération des kératinocytes et l'acanthose, sont encore absentes de ce modèle. En outre, les cellules T issues du sang périphérique sont distinctes des cellules T résidentes de la peau, et en particulier des cellules T résidentes-mémoires (T_{RM}) du derme et de l'épiderme (MUELLER and MACKAY, Nat Rev Immunol., 2016, 16(2), p.79-89). En effet, les cellules T_{RM} possèdent des facteurs de transcription et des marqueurs de surface qui les distinguent des autres cellules T mémoire du sang périphérique. Ainsi, la présence de CD69 à la surface des T_{RM} associée à la sous-expression du gène S1PR1 (Sphingosin-1-phosphate receptor 1) permet leur maintient dans les tissus périphériques comme la peau pendant plusieurs mois, empêchant ainsi leur recirculation dans le sang. La présence de populations CD8⁺ T_{RM} et CD4⁺ T_{RM} a été largement observée dans l'épiderme et le derme de la peau humaine saine; la sous-population CD8⁺CD49a⁺ T_{RM} est impliquée dans la synthèse de perforine et d'IFNγ alors que les cellules CD8⁺CD49a⁻T_{RM} produisent principalement de l'IL-17 (HAIJING et al., Autoimmun Rev., 2018, p. 906-911).

Une autre approche basée sur l'activation des cellules T résidentes dans des biopsies de peau humaine a été décrite dans la demande internationale publiée sous le numéro WO 2014/182655 A1. Toutefois, aucun marqueur protéique caractéristique du psoriasis n'a pu être mis en évidence dans ce modèle. En outre, l'élimination du tissu adipeux sous-cutané conduit à l'obtention de biopsies de peau n'excédant pas 300 µm d'épaisseur. Une telle finesse rend leur manipulation très délicate et ne permet pas de réaliser des injections en sous-cutané.

Il existe par conséquent un réel besoin de développer de nouveaux outils, en particuliers des modèles *ex vivo* de peau humaine inflammée. Ce besoin passe notamment par l'identification des conditions de culture dans lesquelles des échantillons de peau ex *vivo,* appropriés pour une utilisation dans la modélisation de l'inflammation, peuvent être maintenus pendant un temps suffisamment long sans induire de déviation significative du comportement de ces échantillons vis-à-vis des conditions rencontrées *in vivo.*

De manière surprenante, les inventeurs ont mis au point un procédé *in vitro* qui permet de pallier tous ces inconvénients.

### RESUME DE L'INVENTION

La présente invention permet de pallier aux inconvénients précités en fournissant un modèle *ex vivo* de peau inflammée qui reflète de manière plus précise et reproductible l'environnement de la peau inflammée tel qu'observé *in vivo* en contexte physiopathologique.

Ainsi, un premier objet de l'invention concerne un procédé *in vitro* pour obtenir un modèle *ex vivo* de peau inflammée comprenant les étapes suivantes :
a) injecter, dans le derme d'une biopsie de peau saine préalablement prélevée chez un mammifère, une composition comprenant une quantité efficace pour activation des cellules T résidentes du derme d'un anticorps anti-CD3 et d'un anticorps anti-CD28, et optionnellement de l'IL-2, ,
b) incuber la biopsie de peau injectée obtenue à l'étape a) en présence d'une composition comprenant une quantité efficace, pour obtenir la polarisation des cellules T activées à l'étape a) en LTh1 et/ou LTh17 et la synthèse de marqueurs de l'inflammation, d'au moins un mélange d'IL-1β, d'IL-23 et de TGF-β.

Un second objet de la présente invention se rapporte à un modèle *ex vivo* de peau inflammée tel que défini à la revendication 10.

L'utilisation du modèle *ex vivo* de peau inflammée de l'invention dans des méthodes d'identification des composés modulateurs de l'inflammation de la peau offre les avantages de s'appuyer sur un modèle facilement produit, qui reflète plus précisément l'environnement *in vivo* de la peau inflammée, réduisant ainsi les erreurs dans les tests de criblage de composés sous la forme de faux positifs et/ou de faux négatifs.

Ainsi, le procédé selon l'invention vise en outre à évaluer l'efficacité anti-inflammatoire d'un composé et comprend les étapes supplémentaires suivantes :
c) mettre en contact, avec un composé candidat, le modèle *ex vivo* de peau inflammée obtenu à l'issue de l'étape b) et comprenant des cellules T polarisées en LTh1 et/ou LTh17 ;
d) mesurer le niveau d'expression d'au moins deux marqueurs de l'inflammation dans le modèle *ex vivo* de peau inflammée;
e) comparer le niveau d'expression desdits au moins deux marqueurs de l'inflammation obtenus à l'étape d) avec leur niveau d'expression contrôle ;
f) identifier l'efficacité anti-inflammatoire dudit composé candidat lorsque le niveau d'expression desdits au moins deux marqueurs de l'inflammation mesurés à l'étape d) est inférieur à leur niveau d'expression contrôle.

Avec le procédé de l'invention, la recherche d'anti-inflammatoires efficaces pour traiter l'inflammation cutanée, et en particulier le psoriasis, sera facilitée.

Encore un autre objet de l'invention concerne l'utilisation d'un modèle *ex vivo* de peau inflammée obtenu par le procédé de l'invention à des fins de criblages de composés candidats anti-inflammatoires, en particulier de composés candidats susceptibles de traiter le psoriasis.

Enfin, un autre objet de l'invention porte sur un kit permettant la mise en œuvre du procédé de l'invention, ledit kit comprenant une biopsie de peau saine préalablement prélevée chez un mammifère, une composition A comprenant une quantité efficace d'un anticorps anti-CD3 et d'un anticorps anti-CD28, et optionnellement d'IL-2, d'une composition B comprenant au moins un mélange d'IL-1β, d'IL-23 et de TGFβ, et optionnellement un dispositif d'injection par perfusion.

La demande internationale WO 2014/182655 (STIEFEL LABORATORIES INC) décrit une méthode pour cribler un composé capable de moduler la synthèse de cytokines pro-inflammatoires dans la peau humaine, la méthode comprenant la fourniture d'une culture de peau humaine à l'interface liquide-air, son exposition à un composé à tester, l'exposition de cette culture de peau à des conditions d'activation des cellules Th17, la mesure de la synthèse de cytokines pro-inflammatoires et la comparaison des valeurs trouvées avec celle d'une culture de peau n'ayant pas été mise en contact avec le composé à tester.

VAN DEN BOGAARD et al. (J. Invest. Dermatol., vol.134(3), p :719-727, 2014) décrit certes l'activation d'une sous-population de cellules T CD4^{pos} issues du sang périphérique s'effectue pendant 5h au contact de billes coatées avec des anticorps anti-CD3/CD28. Maintenant, ces cellules T ne sont pas des cellules T résidentes du derme.

KOENEN et al. (Blood, vol.112(6), p :2340-2352, 2008) décrit en détail la sélection de la sous-population de cellules T CD4^{pos} issues du sang périphérique et leur activation par les anticorps anti-CD3/CD28 coatés sur des billes placées dans le milieu de culture des cellules T. Maintenant, ces cellules T ne sont pas des cellules T résidentes du derme.

### DESCRIPTION DES DESSINS

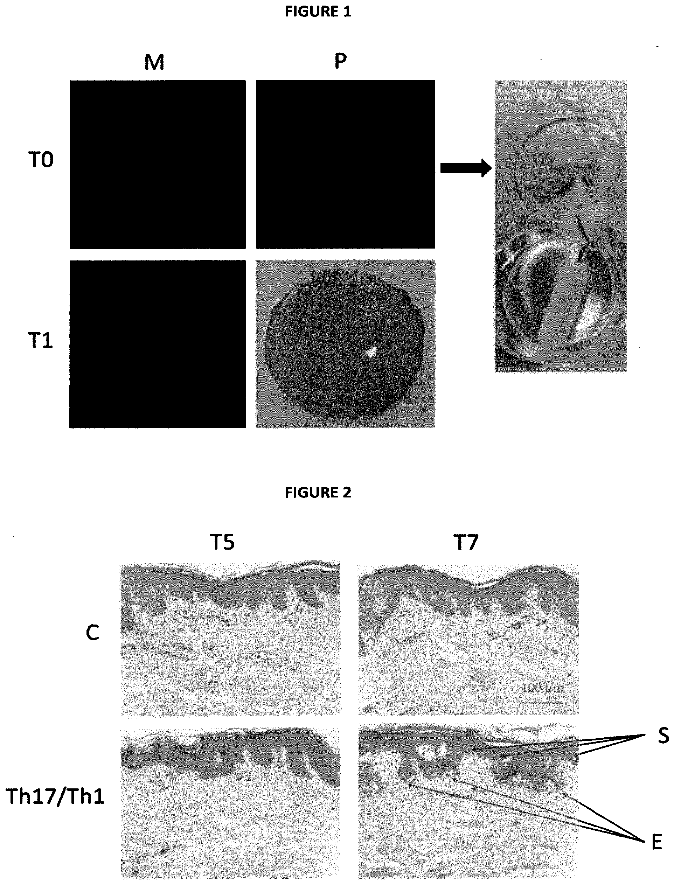

La figure 1 illustre l'injection intradermale manuelle (M) ou par perfusion (P) d'une solution colorée dans une biopsie de peau et sa diffusion en comparant le gradient d'intensité de coloration obtenu par injection manuelle ou par perfusion.
La figure 2 compare les caractéristiques histologiques d'une biopsie de peau saine non traitée (C) ou traitée selon les étapes a) et b) du procédé de l'invention (Th17/Th1). Les observations ont été réalisées 5 jours (T5) ou 7 jours (T7) après le début de l'incubation de la biopsie de peau saine injectée issue de l'étape a) du procédé de l'invention dans la solution de polarisation. Après 7 jours de culture en présence d'une composition comprenant au moins un mélange d'IL-1β, d'IL-23 et de TGFβ, on observe l'apparition de spongiose (S) dans l'épiderme et l'élongation des crêtes épidermales (E).

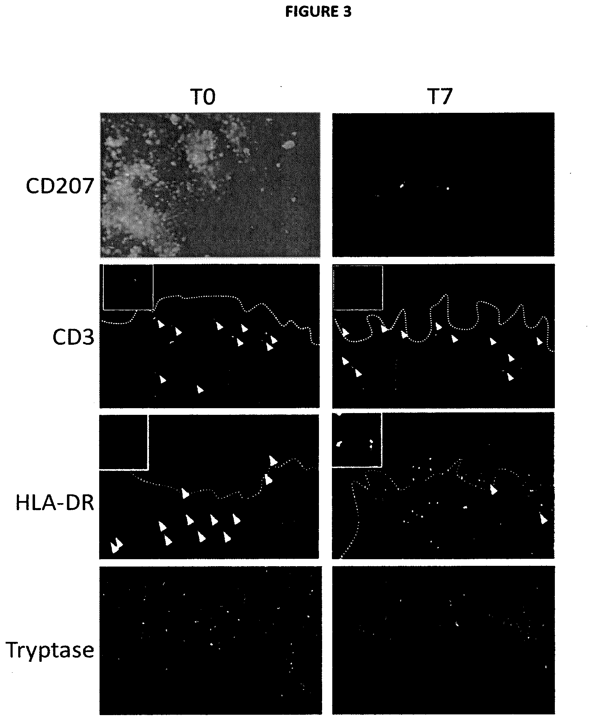

La figure 3 illustre l'analyse histologique de la présence de cellules de Langerhans (CD207), de cellules T résidentes (CD3), de cellules dendritiques (HLA-DR) et de mastocytes (Tryptase) dans une biopsie de peau saine non traitée (T0) et maintenue en culture pendant 7 jours (T7).

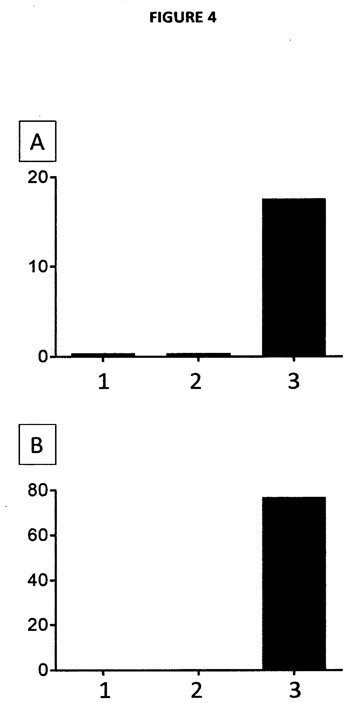

La figure 4 illustre la sécrétion des cytokines pro-inflammatoires IL-17A et IL-22 par le modèle de peau inflammée obtenu par le procédé de l'invention démontrant la présence de LTh17 et LTh1 dans les modèles traités selon les étapes a) et b) du procédé de l'invention (Th17/Th1). Sont comparées une biopsie de peau saine non traitée (1 - NativeSkin^{®}), une biopsie de peau saine cultivée en présence du cocktail de polarisation de l'étape b) du procédé de l'invention (2 - NativeSkin^{®} + pro Th17/Th1), une biopsie de peau saine traitée selon les étapes a) et b) du procédé de l'invention (3 - InflammaSkin^{®}). Les résultats sont exprimés en pg/ml.

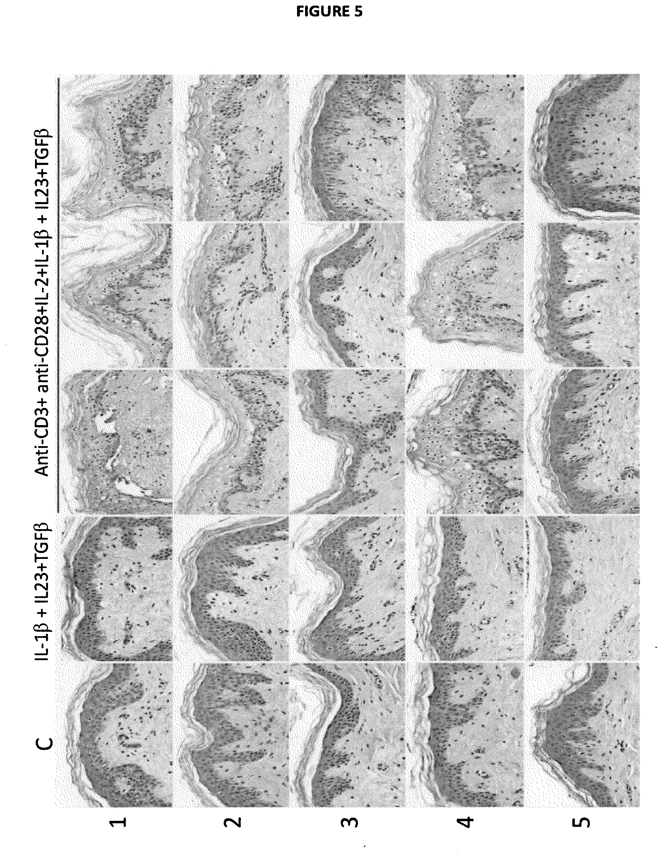

La figure 5 illustre les caractéristiques histologiques d'une biopsie de peau saine non traitée (C) ou traitée selon l'étape b) uniquement (IL-1β+IL-23+TGFβ) ou selon les étapes a) et b) (Anti-CD3+anti-CD28+IL-2+IL-1β+IL-23+TGFβ) du procédé de l'invention et après 7 jours de culture. Ces biopsies ont été non traitées (1), traitées par un gel placébo (2) ou une crème placébo (4), ou traitées par l'un des deux inhibiteurs de l'inflammation betamethasone di-propionate (3) et l'inhibiteur de PhosphoDiEstérase de type 4 (5). Seuls les traitements avec les inhibiteurs de l'inflammation (3 et 5) conduisent à une absence totale d'anomalies histologiques dans l'épiderme suggérant une inhibition de la sécrétion de cytokines pro-inflammatoires et donc de la différenciation des cellules LTh17/LTh1.

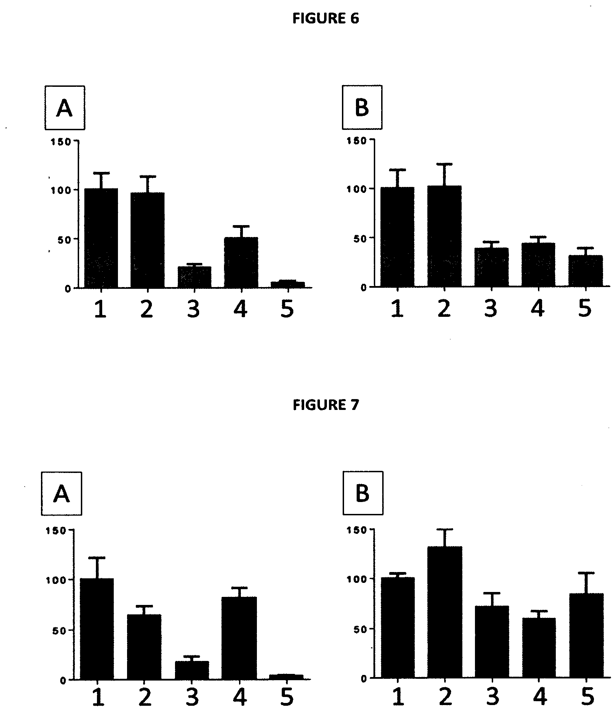

La figure 6 illustre l'inhibition de la synthèse protéique d'IL-17A en traitement prophylactique (A) ou thérapeutique (B). Sont comparés le modèle de peau inflammée obtenu par le procédé de l'invention non traité (1), ou traité par le gel placébo (2), le gel comprenant la betamethasone di-propionate (3), la crème placébo (4) ou la crème comprenant l'inhibiteur de PDE4 (5). Les résultats sont exprimés en % par rapport au modèle de peau inflammée obtenu par le procédé de l'invention non traité (1).
La figure 7 illustre l'inhibition de la synthèse protéique d'IL-22 en traitement prophylactique (A) ou thérapeutique (B). Sont comparés le modèle de peau inflammée obtenu par le procédé de l'invention non traité (1), ou traité par le gel placébo (2), le gel comprenant la betamethasone di-propionate (3), la crème placébo (4) ou la crème comprenant l'inhibiteur de PDE4 (5). Les résultats sont exprimés en % par rapport au modèle de peau inflammée obtenu par le procédé de l'invention non traité (1).

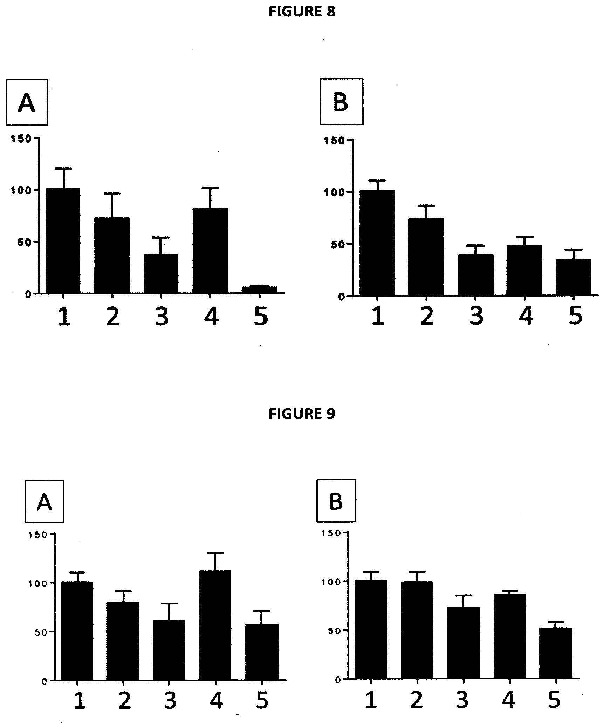

La figure 8 illustre l'inhibition de la synthèse protéique d'IFNγ en traitement prophylactique (A) ou thérapeutique (B). Sont comparés le modèle de peau inflammée obtenu par le procédé de l'invention non traité (1), ou traité par le gel placébo (2), le gel comprenant la betamethasone di-propionate (3), la crème placébo (4) ou la crème comprenant l'inhibiteur de PDE4 (5). Les résultats sont exprimés en % par rapport au modèle de peau inflammée obtenu par le procédé de l'invention non traité (1).
La figure 9 illustre l'inhibition de la synthèse protéique de TNFα en traitement prophylactique (A) ou thérapeutique (B). Sont comparés le modèle de peau inflammée obtenu par le procédé de l'invention non traité (1), ou traité par le gel placébo (2), le gel comprenant la betamethasone di-propionate (3), la crème placébo (4) ou la crème comprenant l'inhibiteur de PDE4 (5). Les résultats sont exprimés en % par rapport au modèle de peau inflammée obtenu par le procédé de l'invention non traité (1).

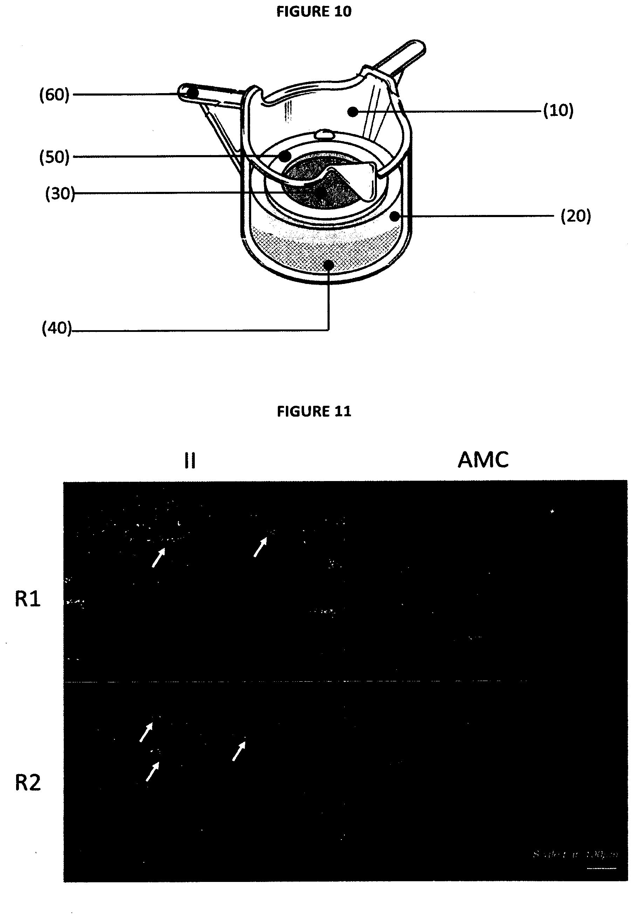

La figure 10 est une représentation schématique du modèle *ex vivo* de peau inflammée obtenu par le procédé de l'invention. Un insert pour culture cellulaire (10) dont le fond est constitué d'une membrane poreuse (40) contient le modèle *ex vivo* de peau inflammée (30) enserré dans une matrice solidifiée (20). Dans un mode de réalisation particulier, l'insert pour culture cellulaire possède des ergots (60) et un anneau constitué par un matériau hydrophobe (50) fixé à la surface épidermique dudit modèle *ex vivo* de peau inflammée.
La figure 11 illustre la capacité d'un anticorps anti-CD3 à incorporer les cellules du derme d'une biopsie de peau humaine. Sont comparés l'injection de l'anticorps anti-CD3e directement dans le derme de la biopsie (Injection Intradermale (II) - panneaux de gauche), et la diffusion de l'anticorps anti-CD3e placé dans le milieu de culture dans lequel la biopsie de peau flotte (Addition au milieu de culture (AMC) panneaux de droite). Deux biopsies distinctes ont été testées (réplicats (R) 1 et 2). Les flèches blanches identifient les cellules du derme ayant incorporé l'anticorps anti-CD3. L'étoile blanche montre la présence de l'anticorps anti-CD3 à la surface de l'épiderme d'une des biopsies de peau (panneau de droite haut).

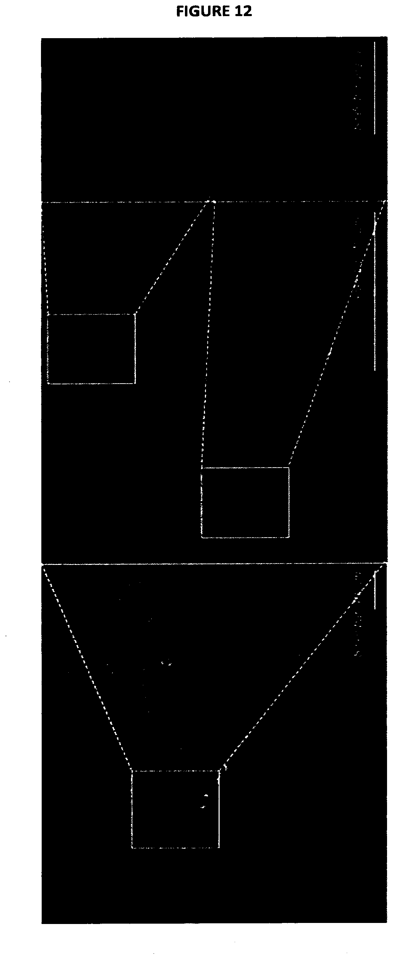

La figure 12 illustre la localisation subcellulaire d'un anticorps anti-CD3 dans les cellules du derme d'une biopsie de peau humaine. L'anticorps anti-CD3e a été injecté directement dans le derme de la biopsie.

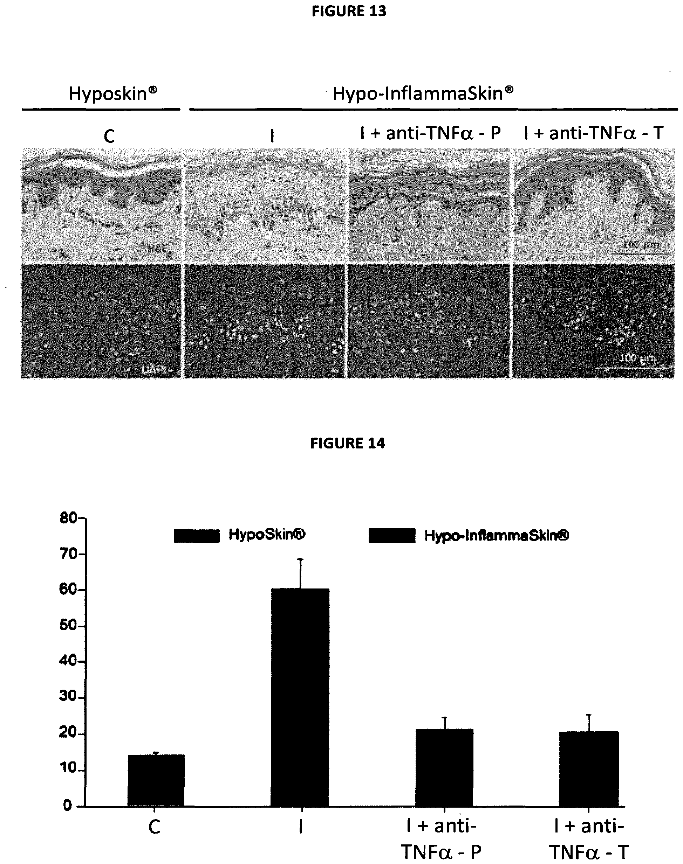

La figure 13 illustre les caractéristiques histologiques d'une biopsie de peau non inflammée (C), inflammée selon le procédé de l'invention (I), inflammée et traitée par injection sous-cutané d'un anticorps anti-TNF-α en traitement prophylactique (I + anti-TNF-α - P) ou en traitement thérapeutique (I + anti-TNF-α -T).
La figure 14 illustre la synthèse protéique d'IL-22 par une biopsie de peau non inflammée (C), inflammée selon le procédé de l'invention (I), inflammée selon le procédé de l'invention et traitée par injection sous-cutané d'un anticorps anti-TNF-α en traitement prophylactique (I + anti-TNF-α - P) ou en traitement thérapeutique (I + anti-TNF-α -T). Les résultats sont exprimés en pg/ml.

### DESCRIPTION DETAILLEE DE L'INVENTION

L'invention a pour but de proposer un nouvel outil pour l'évaluation de l'activité d'un composé sur la diminution de l'inflammation de la peau.

Un premier objet de l'invention concerne un procédé *in vitro* pour obtenir un modèle *ex vivo* de peau inflammée comprenant les étapes suivantes :
a) injecter dans le derme d'une biopsie de peau saine préalablement prélevée chez un mammifère une composition comprenant une quantité efficace pour activation des cellules T résidentes du derme d'un anticorps anti-CD3 et d'un anticorps anti-CD28, et optionnellement d'IL-2,
b) incuber la biopsie de peau injectée obtenue à l'étape a) en présence d'une composition comprenant une quantité efficace, pour obtenir la polarisation des cellules T activées à l'étape a) en LTh1 et/ou LTh17 et la synthèse de marqueurs de l'inflammation, d'au moins un mélange d'IL-1b, d'IL-23 et de TGF-β.

Par « modèle *ex vivo* de peau inflammée», on désigne une biopsie de peau présentant des caractéristiques inflammatoires. L'identification de ces caractéristiques inflammatoires repose sur la détection de l'expression de marqueurs de l'inflammation, au niveau protéique ou génique. Les marqueurs de l'inflammation inclus, mais ne sont pas limités, aux cytokines, aux protéines antibactériennes, aux protéines impliquées dans la biosynthèse des lipides, ou à toute autre molécule dont le niveau d'expression varie entre un état enflammé et un état non enflammé (cf. notamment SERHAN & WARD, Molecular and Cellular Basis of Inflammation, Humana Press). Des interleukines comprennent, mais sans s'y limiter, IL-1A, IL-1B, IL-5, IL-7, IL-8, IL-9, IL-10, IL-12, IL-17A, IL-17C, IL-17F, IL-19, IL-21, IL-22, IL-23, IL-27, IL-31 et IL-33, tout comme les récepteurs d'interleukines y compris, mais sans s'y limiter, IL-10RA, IL-10RB, IL-1R1, IL-5RA (CD125) et IL-9R. Les chimiokines comprennent également, mais sans s'y limiter, C5, Eotaxin, MCP-4, TARC, MCP-1, MIP-3A, CCL22, CCL23, MIP-1B, RANTES, MCP-3, MCP-2, CX3CL1, IL8RA, INP10, L8RB et CXCL3, ainsi que les récepteurs de chimiokines, y compris, mais sans s'y limiter, CCL13 (MCP-4), CCR1, CCR2, CCR3, CCR4, CCR5, CCR6, CCR8, CX3CR1, CXCR1 et CXCR2. En outre, d'autres cytokines sont envisageables, telles que, sans toutefois s'y limiter, MCP-1, GM-CSF, TNFSF5, MCSF, GCSF, TNFSF6, IFNA2, IFNG, TNFA, TNFB, MIF, NAMPT, TRAIL et IFNA1, ainsi que d'autres gènes impliqués dans l'inflammation, y compris, mais sans s'y limiter, CD4, CD40, TNFSF5, FASLG, JAK2, JNK1, NFkB, RAG1, STAT1, les protéines de la famille S100 et la défensine beta.

De préférence, le modèle *ex vivo* de peau obtenu par le procédé de l'invention est considéré comme un modèle *ex vivo* de peau inflammée lorsque les taux d'expression d'au moins deux, de préférence d'au moins trois, et de manière particulièrement préférée d'au moins quatre marqueurs de l'inflammation choisis parmi ceux précités sont supérieurs à ceux observés dans une biopsie de peau saine (non inflammée et/ou n'ayant pas subi les étapes du procédé selon l'invention).

De préférence encore, le modèle *ex vivo* de peau inflammée obtenu par le procédé de l'invention est comparable aux lésions psoriasiques observées chez un patient atteint de psoriasis lorsque, en plus des marqueurs de l'inflammation, on détecte la surexpression d'au moins un gène ou son produit parmi les 50 listés dans le tableau S1 de l'article de Yao et al., PloSOne, 2008, 3(7), e2737 et qui sont incorporés ici par référence.

De manière particulièrement préférée, le modèle *ex vivo* de peau inflammée obtenu par le procédé de l'invention présente des taux d'expression protéique et/ou génique d'au moins trois, de préférence d'au moins quatre, de préférence d'au moins cinq, et de manière particulièrement préférée d'au moins six marqueurs de l'inflammation choisis parmi IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 et CCL20 qui sont supérieurs à ceux observés dans une biopsie de peau saine.

En plus de la présence de marqueurs de l'inflammation ou la surexpression d'autres marqueurs pré-cités, des modifications physiologiques sont également observables sur le modèles *ex vivo* de peau inflammée obtenu par le procédé de l'invention. Parmi celles-ci on observe, mais sans s'y limiter, une spongiose de l'épiderme, une élongation des crêtes épidermales dans le derme, la présence de noyaux pycnotiques et de vacuolisation des cytoplasmes dans les cellules de l'épiderme, voire des détachements suggérant une mort cellulaire provoquée par l'inflammation.

Par « biopsie de peau », on désigne un fragment de peau qui comprend au moins l'épiderme, le derme et les annexes épidermiques. Ce fragment ou biopsie de peau pourra comprendre également une partie du tissu sous-cutané encore appelé hypoderme. L'hypoderme est situé immédiatement sous le derme et forme un coussin de protection séparant la peau des membranes fibreuses entourant les organes plus profonds, muscles, tendons et os. L'hypoderme est constitué d'adipocytes, de nerfs, d'un réseau de capillaires sanguins et lymphatiques, d'un nombre restreint de fibroblastes impliqués dans la synthèse des composants de la matrice extracellulaire, et de cellules immunes tels que des cellules dendritiques et des macrophages. L'hypoderme est divisé en lobules adipeux contenant des adipocytes et séparés par des cloisons conjonctives qui permettent le passage des nerfs et des vaisseaux. Les fonctions de l'hypoderme sont d'isoler, de fournir une réserve d'énergie aux cellules de la peau et d'absorber les contraintes physiques. Par ailleurs, des travaux ont montré le rôle important joué par le transport lymphatique dans l'absorption des médicaments à base de peptides.

De préférence, la biopsie de peau possède une épaisseur variant de 1 millimètre (mm) à 1 centimètre (cm), plus particulièrement de 3 mm à 1 cm, encore plus particulièrement de 3 mm à 8 mm et encore plus particulièrement de 5 mm à 7 mm. Une épaisseur pouvant atteindre 1 cm est obtenue pour des biopsies de peau comprenant l'épiderme, le derme, les annexes épidermiques et l'hypoderme. Dans tous les cas, l'épaisseur de la biopsie de peau n'est pas inférieure à 1 mm.

De préférence, ladite biopsie de peau est prélevée chez un animal, en particulier un mammifère et, de préférence, chez l'humain. Comme biopsies utilisables dans le procédé de l'invention, on peut citer celles issues de déchets chirurgicaux ou d'abattoirs. Par « déchets chirurgicaux », on désigne des échantillons de peaux obtenus par un acte de chirurgie esthétique, notamment après blépharoplastie, rhinoplastie, abdominoplastie ou réduction mammaire. Les techniques de prélèvement de biopsie de peau sont bien connues de l'Homme du métier.

Dans le but d'assurer sa survie *in vitro,* le prélèvement doit avoir été réalisé dans un délai de 1h à moins de 72h, de préférence de 1h à moins de 48h avant l'injection une composition comprenant une quantité efficace d'un anticorps anti-CD3 et d'un anticorps anti-CD28, et optionnellement d'IL-2.

Par « biopsie de peau saine », on désigne un fragment de peau qui ne présente aucun signe d'inflammation perceptible à l'œil (i.e. brèche cutanée, rougeur, gonflement, chaleur ou desquamation excessive, ...) ou n'exprime aucun marqueur de l'inflammation. En outre, il est impératif que la biopsie de peau saine utilisée dans la méthode de l'invention soit issue d'un individu ne présentant aucune pathologie dermatologique, en particulier inflammatoire.

De préférence, la biopsie de peau est de forme cylindrique.

Toutefois, toute autre forme géométrique de biopsie de peau convient également au procédé selon l'invention, en particulier une forme carrée, rectangulaire, ovale, triangulaire, etc....

De préférence, la forme cylindrique possède un diamètre qui varie de 1 mm à 50 mm, plus particulièrement de 5 mm à 20 mm, encore plus particulièrement de 7 mm à 17 mm, et une épaisseur qui varie de 1 mm à 20 mm, plus particulièrement de 2 mm à 15 mm, encore plus particulièrement de 2 mm à 10 mm, et encore plus particulièrement de 2 mm à 5 mm.

Par « derme », on désigne le tissu sous-jacent de l'épiderme qui contient des réseaux vasculaires et nerveux denses ainsi que des annexes kératinisées prolongeant l'épiderme, ce tissu inclut également les follicules pileux, les glandes sébacées et les glandes sudoripares.

Le derme est subdivisé en deux parties qui diffèrent par la composition et l'organisation de leur matrice extracellulaire respective. Le derme papillaire se trouve directement sous l'épiderme. Il s'agit d'un tissu conjonctif lâche constitué de fines fibrilles de collagène de type I et III et des fibres élastiques qui sont orientées perpendiculairement à l'épiderme formant ainsi des structures en forme de chandelles. Le derme réticulaire, placée sous le derme papillaire, est également un tissu conjonctif composé d'un entrecroisement de faisceaux de grosses fibres de collagène et de fibres élastiques qui présentent une orientation préférentiellement parallèle à la surface de la peau. Le derme réticulaire contient moins de collagène de type III que le derme papillaire.

Par « injecter dans le derme », on entend l'injection, l'introduction ou l'inoculation d'une composition directement dans la structure du derme d'une biopsie de peau saine. Cette injection dans le derme s'effectue à l'aide d'un dispositif d'injection, notamment une aiguille creuse, biseautée ou non, dépourvue de volume mort, ou de tout autre dispositif mécanique équivalent connu de l'Homme du métier.

De préférence, l'injection est effectuée dans le derme réticulaire afin d'éviter tout traumatisme de l'épiderme. Dans les biopsies de peau utilisées dans le procédé de l'invention, la présence sous le derme des annexes épidermiques et optionnellement de l'hypoderme, leur confère une épaisseur limitant le risque de déchirure lors d'une injection.

Une injection dans le derme des biopsies du document WO 2014/182655 A1 présenterait un risque de déchirure accrue dans la mesure où ces biopsies dépourvue d'hypoderme et dermatomées ont une épaisseur de 750 µm (SMITH et al., PLoS One. 2016 Feb 12;11(2):e0147979).

Le principal avantage d'une injection d'une composition directement dans la structure du derme d'une biopsie de peau saine est de permettre une diffusion plus homogène et plus rapide de cette composition dans tous les tissus de la biopsie, et par conséquent une meilleure activation des cellules T résidentes du derme. De telles performances ne sauraient être obtenues par simple diffusion passive de la composition comme cela est utilisé dans le document WO 2014/182655 A1.

De préférence encore, l'étape d'injection est réalisée à température ambiante.

Le volume de la composition injectée dans le derme varie en fonction de la taille ou de la surface de la biopsie de peau saine, en particulier de son diamètre si celle-ci est de forme cylindrique.

De préférence, lorsque le diamètre de la biopsie de peau saine est inférieur ou égal à 8 mm le volume de composition injectée est inférieur ou égal 10 µl.

De préférence, lorsque le diamètre de la biopsie de peau saine est inférieur ou égal à 15 mm le volume de composition injectée est inférieur ou égal 35 µl.

De préférence, le volume de la composition injectée est d'au moins 5µl.

Il est important, lors de l'injection, de ne pas endommager l'épiderme. Un tel résultat ne peut être obtenu que si la biopsie de peau possède une épaisseur d'au moins 1 mm, de préférence au moins 2 mm, et de préférence encore au moins 3 mm.

De préférence, l'injection de la composition peut être réalisée manuellement ou par perfusion.

Selon un mode de réalisation particulier, l'injection de la composition comprenant une quantité efficace d'un anticorps anti-CD3 et d'un anticorps anti-CD28, et optionnellement de l'IL-2 dans le derme de la biopsie de peau saine est réalisée manuellement.

L'injection de la composition comprenant un anticorps anti-CD3 et un anticorps anti-CD28, et optionnellement de l'IL-2 dans le derme de la biopsie de peau saine permet une incorporation des anticorps anti-CD3 et anti-CD28, et optionnellement d'IL-2, dans les cellules du derme.

Selon un autre mode de réalisation particulier, l'injection de la composition comprenant une quantité efficace d'un anticorps anti-CD3 et d'un anticorps anti-CD28, et optionnellement de l'IL-2 dans le derme de la biopsie de peau saine est réalisée par perfusion, selon un débit variant de 4 à 12 µL / heure. Dans ce mode de réalisation particulier, l'injection peut durer plusieurs heures. Afin d'assurer un débit constant, l'utilisation d'une pompe osmotique ou de tout autre système de diffusion liquide contrôlée est envisageable.

Contrairement à une injection manuelle, l'injection par perfusion permet une diffusion plus homogène de la composition dans le derme de la biopsie de peau. Par « diffusion plus homogène », on entend une réduction du gradient de concentration des composés de la composition à partir du point d'injection vers le bord (i.e. diamètre externe) de la biopsie. En outre, l'injection par perfusion assure une meilleure reproductibilité.

Par « anticorps anti-CD3 », on désigne tout anticorps ou fragment de celui-ci capable de lier spécifiquement l'antigène CD3. CD3 est un complexe protéique membranaire présent à la surface des cellules T naïves et est constitué d'une chaîne γ, d'une chaîne δ et deux chaînes ε. En s'associant avec le récepteur des cellules T (TCR) et avec une chaîne complémentaire ζ, CD3 permet la formation du complexe TCR capable de transduire un signal intracellulaire. Il existe des formes commerciales de l'anticorps anti-CD3, par exemple le clone UCHT1 (THERMO FISHER 16-0038-81, MERCK MILLIPORE CBL150), ou bien le clone APA1/1 (MERCK MILLIPORE 05-785, THERMO FISHER MA1-10182).

De préférence, la concentration en anticorps anti-CD3 dans la composition injectée à l'étape a) varie de 10 ng/µl à 100 ng/µl, de préférence de 20 ng/µl à 80 ng/µl, et de manière particulièrement préférée de 30 ng/µl à 70 ng/µl.

Selon un mode de réalisation particulier, la concentration en anticorps anti-CD3 dans la composition injectée à l'étape a) est égale à 50 ng/µl.

Par « anticorps anti-CD28 », on désigne tout anticorps ou fragment de celui-ci capable de lier spécifiquement l'antigène CD28. CD28 est une protéine de co-stimulation présente notamment à la surface des cellules T et impliquée dans leur survie, leur expansion clonale, leur activité métabolique ainsi que la production d'IL-2. Il existe des formes commerciales de l'anticorps anti-CD28, par exemple le clone 15E8 (MILTENYI BIOTEC 130-093-375) ou le clone CD28.6 (THERMO FISHER 16-0288-81)

De préférence, la concentration en anticorps anti-CD28 dans la composition injectée à l'étape a) varie de 10 ng/µl à 100 ng/µl, de préférence de 20 ng/µl à 80 ng/µl, et de manière particulièrement préférée de 30 ng/µl à 70 ng/µl.

Selon un mode de réalisation particulier, la concentration en anticorps anti-CD28 dans la composition injectée à l'étape a) est égale à 50 ng/µl.

Selon un mode de réalisation plus particulier, l'anticorps anti-CD3 et l'anticorps anti-CD28 sont remplacés par un unique anticorps bispécifique possédant un premier fragment ScFV spécifiquement dirigé contre CD3 et un second fragment ScFV spécifiquement dirigé contre CD28. Cet anticorps bispécifique possède des activités de liaison aux deux antigènes CD3 et CD28 et des fonctions d'activation des cellules T résidentes comparables à celles des anticorps monospécifiques anti-CD3 et anti-CD28.

Par « IL-2 », on désigne l'interleukine 2 qui est une cytokine agissant sur la mitose et par conséquent la prolifération des cellules portant le récepteur à l'IL-2, en particulier les cellules T helper.

De préférence, la concentration en IL-2 dans la composition injectée à l'étape a) varie de 1 ng/ml à 20 ng/ml, de préférence de 2 ng/ml à 15 ng/ml, et de manière particulièrement préférée de 4 ng/ml à 12 ng/ml.

Selon un mode de réalisation particulier, la concentration IL-2 dans la composition injectée à l'étape a) est égale à 10 ng/ml.

Dans les heures qui suivent l'injection de la composition comprenant un anticorps anti-CD3 et un anticorps anti-CD28, et optionnellement de l'IL-2, dans le derme de la biopsie de peau saine, les sous-populations de cellules T CD4⁺ et/ou CD8⁺ naïves ou mémoires résidentes du derme sont activées, et potentiellement amplifiées. Ces cellules étant en nombre restreint et réparties aléatoirement dans le derme, il est important que la composition diffuse de manière homogène dans tout le derme, et optionnellement dans l'épiderme de la biopsie.

Par « quantité efficace », on désigne une quantité suffisante pour obtenir l'effet attendu. Cette notion de quantité efficace est applicable à l'anticorps anti-CD3, à l'anticorps anti-CD28 et également à l'IL-2. Au sens de la présente invention et en lien avec l'étape a), « l'effet attendu » est l'activation des cellules T résidentes du derme. Cet effet est amélioré lorsque l'injection de la composition est effectuée par perfusion.

L'activation des cellules T résidentes du derme peut être mise en évidence directement en utilisant des marqueurs d'activation, tel que par exemple, mais sans s'y limiter CD69.

Les quantités efficaces d'anticorps anti-CD3, anticorps anti-CD28 et IL-2 varie en fonction de la taille de la biopsie de peau.

A titre d'exemple de quantité efficace, on peut évoquer l'utilisation des volumes d'injection en lien avec les concentrations listées précédemment.

Selon un mode de réalisation particulier, les quantités efficaces d'anticorps anti-CD3, d'anticorps anti-CD28 et d'IL-2 dans la composition injectée dans le derme de la biopsie de peau saine à l'étape a) du procédé de l'invention sont respectivement de 500 ng, 500 ng et 0,1 ng pour une biopsie de diamètre égal à 8 mm.

Selon un autre mode de réalisation particulier, les quantités efficaces d'anticorps anti-CD3, d'anticorps anti-CD28 et d'IL-2 dans la composition injectée dans le derme de la biopsie de peau saine à l'étape a) du procédé de l'invention sont respectivement de 1750 ng, 1750 ng et 0,35 ng pour une biopsie de diamètre égal à 15 mm.

Selon un mode de réalisation particulier, le procédé *in vitro* selon l'invention comprend, préalablement à l'étape b), une étape a') de dépôt de la biopsie de peau injectée obtenue à l'issue de l'étape a) sur une matrice liquide apte à se solidifier, ladite matrice étant elle-même contenue dans un insert pour culture cellulaire dont le fond est constitué d'une membrane poreuse et ledit insert étant disposé dans un container ou puits, de sorte à permettre, une fois la matrice solidifiée, le maintien de l'intégrité 3D de la biopsie de peau.

Par « matrice liquide apte à se solidifier », on désigne toute solution liquide comprenant au moins un composé ou une composition spécifique dont la concentration dans ladite solution liquide est telle que, lors de la mise en œuvre de conditions appropriées, notamment des conditions particulières de température, la solution liquide prend une consistance de type solide ou gélifiée. Ledit composé ou une composition spécifique peut être d'origine animale, végétale ou synthétique, sa nature et sa concentration sont déterminées en fonction des caractéristiques physico-chimiques souhaitées de la matrice lorsqu'elle est solidifiée, notamment la souplesse et la résistance de la matrice.

Selon un mode de réalisation du procédé selon l'invention, à l'étape a'), ladite matrice liquide apte à se solidifier est choisie parmi toute solution liquide, de préférence nutritive, capable de se solidifier ou de se gélifier dans des conditions particulières compatibles avec la survie et la culture de cellules de peau qui composent ladite biopsie de peau, de préférence choisie parmi le plasma sanguin ou une solution dérivée de plasma sanguin, notamment dilué en tampon physiologique à au maximum 10 %, de préférence à au moins 20 %, au moins 30 % et 40 %, une solution de fibrinogène, une solution de collagène, de la gélatine, les gels polymériques synthétiques, naturels tels que les gels d'agarose, en particulier les gels d'agarose ou d'agar-agar à faible/bas points de fusion, les gels d'amidon ou de polysaccharides, ou un de leurs mélanges.

Les matrices aptes à se solidifier et les méthodes pour y placer les biopsies de peau sont détaillées dans la demande de brevet Européen n° EP 2 882 290 A1 et sont incorporées ici par référence.

La matrice apte à se solidifier a pour but de permettre la mise en culture de la biopsie de peau injectée obtenue à l'issue de l'étape a) du procédé de l'invention et une manipulation plus aisée de celle-ci. Par « mise en culture », on désigne le maintien de l'état physiologique et morphologique de la biopsie de peau, c'est-à-dire de l'intégralité des tissus et des cellules qui la composent.

La matrice apte à se solidifier a pour but de permettre également la conservation et/ou le transport de la biopsie de peau dans laquelle les cellules T résidentes ont été activées par le procédé selon l'invention.

La nature de la matrice apte à se solidifier doit permettre le maintien en vie des cellules de la biopsie de peau, c'est-à-dire n'avoir aucun effet cytotoxique et posséder une température de solidification/polymérisation à température ambiante (i.e. de 15°C à 25°C).

Dans un mode de réalisation particulier, la matrice apte à se solidifier est une solution de fibrinogène humain.

Selon un mode de réalisation particulier, le procédé *in vitro* selon l'invention comprend, préalablement à l'étape a'), une étape a") de fixation, à la surface épidermique de la biopsie de peau injectée obtenue à l'issue de l'étape a), d'un anneau, ou disque perforé en son centre, constitué par un matériau hydrophobe, le diamètre extérieur dudit anneau étant supérieur au diamètre de la surface épidermique de la biopsie de peau et le diamètre intérieur dudit anneau étant inférieur au diamètre de la surface épidermique de la biopsie de peau.

De préférence, le matériau hydrophobe de l'anneau est un matériau non toxique pour la peau, il peut être un polymère de paraffine, de type Parafilm^{®} (SIGMA), ou un polymère de silicone. Selon un mode particulier, l'anneau est préparé à partir d'un film de matière hydrophobe, par la perforation de ce film selon les dimensions désirées.

L'épaisseur de l'anneau est de préférence comprise entre 0,1 mm et 2 mm, de préférence entre 0,1 et 1 mm, plus préférentiellement entre 0,1 et 0,5 mm, et encore plus préférentiellement entre 0,12 et 0,2 mm.

Ledit anneau peut être constitué par un matériau opaque ou translucide. Selon un mode de réalisation particulier, l'anneau est constitué par un matériau opaque.

Selon un mode de réalisation plus particulier, ledit anneau est fixé à la surface épidermique de la biopsie à l'aide de colle, de préférence ajoutée à la surface inférieure de l'anneau. Ladite colle peut-être choisie parmi tout type de matériau non toxique pour la peau et ayant pour effet l'adhésion de l'anneau à la peau, ce matériau pouvant être du silicone. De préférence, ladite colle est hydrophobe.

Les matériaux, colles et modes de fixation de cet anneau hydrophobe sont détaillées dans la demande de brevet Européen n° EP 2 882 290 A1 et sont incorporés ici par référence.

Par « incuber la biopsie de peau injectée obtenue à l'étape a) », on désigne l'étape qui consiste à placer la biopsie de peau préalablement injectée au niveau du derme avec la composition comprenant une quantité efficace d'anticorps anti-CD3 et d'un anticorps anti-CD28, et optionnellement de l'IL-2, dans un milieu de culture contenant tous les éléments nécessaires à sa survie. L'étape b) est donc effectuée en mettant en contact la biopsie de peau obtenue à l'issu de l'étape a) avec un milieu de culture contenant tous les éléments nécessaires à sa survie.

Lors de cette étape b), les cellules T résidentes du derme qui ont été activées à l'issue de l'étape a) sont en outre polarisées en cellules LTh1 et/ou LTh17. Par « polariser », on désigne la différenciation des cellules T résidentes activées vers les lignages de lymphocytes T helper LTh1 et/ou LTh17.

Par « cellules Th1 », on désigne la sous-population de cellules T issue de la différenciation des cellules T CD4+ naïves sous l'effet des cytokines IL-12 et IFN-yet qui possèdent un profil de synthèse de cytokines spécifiques de cette sous-population, à savoir l'IFN-γ, le TNF-α et l'IL-2.

Par « cellules Th17 », on désigne la sous-population de cellules T issue de la différenciation des cellules T CD4⁺ et/ou CD8⁺ naïves ou mémoires sous l'effet des cytokines TGF-β, IL-6, IL-1 et IL-23 et qui possèdent un profil de synthèse de cytokines spécifiques de cette sous-population, à savoir l'IL-17 et l'IL-22.

La mesure de la production de cytokines spécifiques de ces deux sous-populations cellulaires Th1 et/ou Th17 est directement corrélée aux nombres de ces cellules.

Cette différenciation est induite en ajoutant dans le milieu de culture des biopsies de peau saine ayant préalablement été injectées comme défini à l'étape a) du procédé de l'invention, une composition comprenant une quantité efficace d'au moins un mélange d'interleukine-1beta (IL-1β), d'interleukine-23 (IL-23) et du facteur de croissance transformant beta (TGF-β).

Par « interleukine-1beta », on désigne une protéine d'environ 30 KDa résultant de la protéolyse par la caspase 1 d'un précurseur naturellement sécrété par les macrophages et les cellules dendritiques en réponse à une agression bactérienne. Cette cytokine est un médiateur important de la réponse inflammatoire et est impliquée dans de nombreuses activités cellulaires incluant la prolifération, la différenciation et l'apoptose.

Par « interleukine-23 », on désigne une protéine hétérodimérique d'environ 60 KDa composée d'une sous-unité p19 spécifique de l'IL-23 et d'une sous-unité p40 commune à l'IL12. En se fixant sur un récepteur hétérodimérique IL12RB1 et IL23R présent à la surface de certaines sous-populations de cellules T, cette cytokine permet la stimulation des cellules T mémoires conduisant à la synthèse de cytokines pro-inflammatoires.

Par « TGF-β », on désigne une protéine dimérique de 25 KDa correspondant à la forme mature d'une cytokine synthétisée sous forme de précurseur. Cette cytokine est sécrétée par un grand nombre de cellules, dont les macrophages, et exercent des effets pléïotropiques sur les cellules qui s'étendent de la prolifération à l'apoptose. Le TGF-β joue également un rôle très important dans la régulation de l'inflammation.

Par « quantité efficace », au sens de la présente invention et en lien avec l'étape b), on entend une quantité efficace d'IL-1β, d'IL-23 et de TGF-β pour obtenir la polarisation des cellules T activées à l'étape a) en LTh1 et/ou LTh17 et la synthèse de marqueurs de l'inflammation.

De préférence, la concentration en IL-1β dans le mélange de la composition utilisée à l'étape b) du procédé de l'invention varie de 1 ng/ml à 50 ng/ml, de préférence de 5 ng/ml à 30 ng/ml, et de manière particulièrement préférée de 7 ng/ml à 15 ng/ml.

Selon un mode de réalisation particulier, la concentration en IL-1β dans le mélange de la composition utilisée à l'étape b) du procédé de l'invention est de 10 ng/ml.

De préférence, la concentration en IL-23 dans le mélange de la composition utilisée à l'étape b) du procédé de l'invention varie de 10 ng/ml à 100 ng/ml, de préférence de 20 ng/ml à 80 ng/ml, et de manière particulièrement préférée de 30 ng/ml à 60 ng/ml.

Selon un mode de réalisation particulier, la concentration en IL-23 dans le mélange de la composition utilisée à l'étape b) du procédé de l'invention est de 50 ng/ml.

De préférence, la concentration en TGF-β dans le mélange de la composition utilisée à l'étape b) du procédé de l'invention varie de 1 ng/ml à 50 ng/ml, de préférence de 5 ng/ml à 30 ng/ml, et de manière particulièrement préférée de 7 ng/ml à 15 ng/ml.

Selon un mode de réalisation particulier, la concentration en TGF-β dans le mélange de la composition utilisée à l'étape b) du procédé de l'invention est de 10 ng/ml.

Selon un mode de réalisation plus particulier, les concentrations d'IL-1β, d'IL-23 et de TGF-β dans le mélange de la composition utilisée à l'étape b) du procédé de l'invention sont respectivement de 10 ng/ml, 50 ng/ml et 10 ng/ml.

L'étape b) du procédé de l'invention est réalisée à 37°C dans un incubateur à CO₂ pendant un laps de temps suffisamment long pour permettre la polarisation des cellules TCD4+ activées du derme en cellules Th1 et/ou Th17.

De préférence, l'étape b) d'incubation de la biopsie de peau injectée obtenue à l'étape a) en présence d'une composition comprenant au moins un mélange d'IL-1β, IL-23 et de TGFβ est effectuée pendant une durée d'au moins 3 jours, de préférence comprise entre 3 et 10 jours.

De préférence, la durée d'incubation de l'étape b) du procédé de l'invention ne peut être inférieure à 5 jours.

Selon un mode de réalisation particulier, la durée de l'étape b) d'incubation de la biopsie de peau injectée obtenue à l'étape a) en présence d'une composition comprenant au moins un mélange d'IL-1β, IL-23 et de TGF-β est de 7 jours.

De préférence, la composition comprenant au moins un mélange d'IL-1β, IL-23 et de TGF-β utilisée à l'étape b) du procédé de l'invention est renouvelée fréquemment dans le but de maintenir une concentration constante des composants du mélange, leur demi-vie en solution étant de quelques minutes à quelques heures.

Selon un mode de réalisation particulier, la composition comprenant au moins un mélange d'IL-1β, IL-23 et de TGF-β utilisée à l'étape b) du procédé de l'invention est renouvelée quotidiennement pendant toute la durée de cette étape.

Selon un autre mode de réalisation particulier, le procédé selon l'invention vise en outre à évaluer l'efficacité anti-inflammatoire d'un composé et comprend les étapes supplémentaires suivantes :
c) mettre en contact, avec un composé candidat, le modèle de peau inflammée obtenu à l'issue de l'étape b) comprenant des cellules T polarisées en LTh1 et/ou LTh17 ;
d) mesurer le niveau d'expression d'au moins deux marqueurs de l'inflammation dans le modèle de peau inflammée;
e) comparer le niveau d'expression desdits au moins deux marqueurs de l'inflammation obtenus à l'étape d) avec leur niveau d'expression contrôle ;
f) identifier l'efficacité anti-inflammatoire dudit composé candidat lorsque le niveau d'expression desdits au moins deux marqueurs de l'inflammation mesuré à l'étape d) est inférieur à leur niveau d'expression contrôle.

Par « efficacité anti-inflammatoire », on désigne la capacité d'un composé à réduire l'inflammation, comme par exemple, mais sans s'y limiter, à réduire la production de cytokines pro-inflammatoires, à promouvoir la synthèse de cytokines anti-inflammatoires, à moduler la prolifération des cellules du système immunitaires.

Par « composé candidat », on désigne tout composé chimique synthétique ou naturel choisi pour son utilisation potentielle comme modulateur de l'inflammation. Les composés candidats peuvent être purifiés ou mélangés. Les composés candidats incluent des petites molécules, des macromolécules et des polymères. Dans certains modes de réalisation, un composé candidat peut être une molécule produite par synthèse chimique ou contenu dans une bibliothèque combinatoire. Dans certains modes de réalisation, un composé candidat peut être une molécule ou une macromolécule dont la structure est conçue par ordinateur ou une analyse tridimensionnelle. Le composé candidat comprend également des extraits bruts ou purifiés de sources organiques (par exemple, des extraits animaux, des extraits végétaux et des lysats microbiens). Les composés candidats utilisés dans la mise en pratique du procédé de l'invention peuvent être combinés avec un tampon inerte (par exemple, une solution saline) ou un solvant approprié (par exemple, le diméthylsulfoxyde (DMSO), des alcools tels que le méthanol et l'éthanol, des solutions aqueuses ou tout autre composé entrant dans des compositions cosmétiques.

Par « marqueur de l'inflammation », on désigne un élément exprimable, ou l'élément exprimé par celui-ci, qui présente un changement de niveau d'expression dans une cellule, un tissu ou un organe qui est enflammé par rapport à une cellule, un tissu ou un organe qui n'est pas enflammé. Certains marqueurs de l'inflammation présentent des différences statistiquement significatives (p≤0,10) dans le niveau d'expression dans les cellules, tissus ou organes enflammés par rapport aux cellules, tissus ou organes non enflammés. Par exemple, un « marqueur de l'inflammation » peut être un gène, un fragment de gène ou une région codante exprimée à un taux différent dans des cellules contenues dans un tissu enflammé par rapport à des cellules du même type contenues dans un tissu similaire qui n'est pas enflammé. Un « marqueur de l'inflammation » peut correspondre à une production d'ARN (par exemple, un ARNm) ou une production de protéines, mesurée par la présence physique de la protéine ou par une activité caractéristique de cette protéine. Une liste de marqueurs de l'inflammation figure plus haut dans la description.

En fonction de la nature du marqueur de l'inflammation (ARNm ou protéine), on appliquera les méthodes de détection bien connues de l'Homme du métier. Parmi celles-ci, mais sans s'y limiter, on peut citer les western-blots ou les tests de type ELISA pour la détection des protéines. L'expression d'ARNm des gènes d'intérêt peut être déterminée à l'aide de techniques de microarray, d'ARN-Seq ou de séquençage NextGen. Il est également envisageable de mesurer les marqueurs de l'inflammation directement dans le milieu de culture du modèle de peau inflammée, dans tout ou partie du modèle de peau inflammée, ou spécifiquement dans le derme ou l'épiderme ou encore dans certains sous-types cellulaires du derme et/ou de l'épiderme.

La mise en contact du composé candidat avec le modèle de peau inflammée lors de l'étape c) peut s'effectuer par voie topique ou directement dans le milieu de culture. Dans le cas d'une application topique, la surface d'épiderme du modèle *ex vivo* de peau inflammée obtenu à l'issue des étapes a) et b) du procédé de l'invention est séchée (i.e élimination d'éventuelles traces de liquides sur l'épiderme) avant l'application du composé candidat.

Il est également envisageable que cette étape de mise en contact du composé candidat avec le modèle de peau inflammée lors de l'étape c) s'effectue par injection en sous-cutané. Un tel mode de réalisation est particulièrement indiqué pour les composés candidats ayant une masse moléculaire élevée, sont très hydrophiles ou sont facilement dégradés. A titre d'exemple, de tels composés peuvent être des composés candidats de nature protéique, en particulier des anticorps.

De préférence, l'étape c) de mise en contact du composé candidat avec le modèle de peau inflammée peut avoir une durée variant de quelques minutes à quelques heures, voir de quelques jours. Suivant la nature, la stabilité, la solubilité ou toute autre propriété physico-chimique du composé candidat, cette mise en contact peut également être renouvelée quotidiennement pendant une durée totale de 7 jours.

De préférence, lors de l'étape d) du procédé de l'invention, on procèdera à la mesure du niveau d'expression d'au moins trois, de préférence d'au moins quatre, et de manière particulièrement préférée d'au moins cinq marqueurs de l'inflammation.

Les marqueurs de l'inflammation sont tels que définis précédemment.

Selon un mode de réalisation particulier, l'étape d) comprend également la mesure du niveau d'expression d'au moins trois, de préférence d'au moins quatre, notamment d'au moins cinq et, de manière particulièrement préférée, d'au moins six marqueurs de l'inflammation choisis parmi IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 et CCL20.

Le niveau d'expression contrôle peut être mesuré dans le modèle de peau inflammée obtenu par le procédé de l'invention à l'étape b), et avant l'étape c) de sa mise en contact avec le composé candidat.

Le niveau d'expression contrôle peut également être mesuré dans un ou plusieurs échantillons de peau inflammée, en particulier de lésions psoriasiques, prélevés chez un ou plusieurs patients atteints de pathologies inflammatoires de la peau, en particulier de psoriasis.

De préférence, à l'issue de l'étape f) du procédé de l'invention, on sélectionnera les composés candidats qui sont associés à une diminution des niveaux d'expression protéique et/ou génique des au moins deux marqueurs de l'inflammation d'au moins 10 %, de préférence d'au moins 30%, de préférence d'au moins 50% et de manière particulièrement préférée d'au moins 80%.

Un tel procédé permet d'identifier les composés ayant un effet thérapeutique sur une inflammation déjà en place.

Un tel effet ne peut pas être mesuré d'après le modèle développé dans le document WO 2014/182655 A1. En effet, il semble que ce modèle ne puisse être analysé qu'au mieux 48h après l'activation des cellules T résidentes du derme.

Il est également envisageable de tester l'efficacité d'un composé à empêcher la mise en place de l'inflammation.

Selon encore un autre mode de réalisation particulier, les étapes b) et c) du procédé de l'invention sont réalisées simultanément de sorte à empêcher la mise en place de l'inflammation dans l'hypothèse où le composé candidat testé possède un effet anti-inflammatoire.

Il est également envisageable de tester l'efficacité prophylactique d'un composé.

Dans ce cas, l'ordre des étapes a), b) et c) du procédé de l'invention est inversé de sorte à prévenir la mise en place de l'inflammation. Dans ce mode de réalisation, la mise en contact du composé candidat avec le modèle de peau a lieu avant les étapes a) d'activation et b) de polarisation des cellules T résidentes du derme.

Le procédé de l'invention peut également être utilisé en complément d'autres tests de criblage de composés candidats modulateurs de l'inflammation de la peau, comme par exemple, mais sans s'y limiter, des tests *in vitro* sur des sous-populations cellulaires de la peau, des tests enzymatiques, ou encore des test *ex vivo* réalisés sur des biopsies de peau issues de patients présentant une pathologie inflammatoire de la peau (i.e. des dermatites, des lésions psoriasiques, ...).

Un autre objet de l'invention concerne un modèle *ex vivo* de peau inflammée comprenant un derme, un épiderme, des annexes épidermiques, un hypoderme et des cellules Th1 et/ou Th17 et est caractérisé par l'expression au niveau protéique d'au moins deux marqueurs de l'inflammation et d'au moins trois, notamment d'au moins quatre, de préférence d'au moins cinq et, de manière particulièrement préférée, d'au moins six marqueurs de l'inflammation choisis parmi IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 et CCL20.

Afin d'optimiser sa maintenance en survie *ex vivo,* sa manipulation et son transport, ledit modèle *ex vivo* de peau inflammée peut être placé dans une matrice solidifiée qui maintient l'épiderme en contact avec l'air, ladite matrice étant contenue dans un insert pour culture cellulaire.

Un autre objet de l'invention concerne un insert pour culture cellulaire (10), apte à être contenu dans un container ou un puits de boite de culture, et dont le fond est constitué d'une membrane poreuse (40), ledit insert contenant le modèle *ex vivo* de peau inflammée (30) enserré dans une matrice solidifiée (20), ladite matrice étant en contact avec le bord interne de l'insert et la membrane poreuse, l'épiderme du modèle *ex vivo* de peau inflammée étant en contact avec l'atmosphère et le derme et les annexes épidermiques du modèle *ex vivo* de peau inflammée étant immergés dans la matrice solidifiée.

Selon un mode de réalisation particulier, ledit insert pour culture cellulaire dont le fond est constitué d'une membrane poreuse est un insert en forme de nacelle (i.e. de U), de préférence dont le diamètre est compris entre 5 et 40 mm, de manière plus préférée entre 9,5 et 30 mm.

Selon un autre mode de réalisation particulier, ledit insert pour culture cellulaire (10), apte à être contenu dans un container ou un puits de boite de culture, et dont le fond est constitué d'une membrane poreuse (40), ledit insert contenant la biopsie de peau saine injectée obtenue à l'issue de l'étape a) du procédé de l'invention (30) enserrée dans une matrice solidifiée (20), ladite matrice étant en contact avec le bord interne de l'insert et la membrane poreuse, l'épiderme de la biopsie de peau étant en contact avec l'atmosphère et le derme et les annexes épidermiques de la biopsie de peau étant immergés dans la matrice solidifiée. Optionnellement, la matrice solidifiée contient également au moins le mélange d'IL-1β, d'IL-23 et de TGF-β.

De préférence encore, ledit insert pour culture cellulaire est un insert suspendu ou sur pilotis, de préférence suspendu grâce à la présence d'ergots (60).

Selon un autre mode de réalisation particulier, un anneau hydrophobe (50) est fixé à la surface épidermique du modèle *ex vivo* de peau inflammée.

De préférence encore, ladite membrane poreuse est une membrane d'une porosité permettant d'éviter à la matrice liquide de couler au travers de la membrane avant sa solidification, de préférence variant entre 0,4 à 8 µm, de manière préférée encore entre 0,4 et 1, 5 µm, entre 0,8 µm et 1,2 µm étant la porosité préférée.

De préférence encore, ladite membrane poreuse est une membrane choisie en polyéthylène téréphtalate (PET), nitrocellulose ou polycarbonate.

Parmi les inserts pour culture cellulaire, on peut citer ceux fournis en particulier par la société NUNC (Roskilde, Danemark), BD Falcon (BECTON DICKINSON France SAS, 38801 Le Pont-De-Claix, France, Millicell^{®} (EMD MILLIPORE CORPORATION, Billerica, MA, USA) ou Costar^{®} (Grosseron SAS, 44819 Saint-Herblain France), par exemple les inserts avec membrane en polycarbonate, en PET ou nitrocellulose pré-emballés dans des plaques multi-puits de 6, 8, 12 et 24 puits et dont la porosité de la membrane peut varier entre 0,4 et 8 µm, les boites de 8 puits et/ou avec une porosité de membrane de 0,8 µm à 1 µm et/ou en PET étant les plus préférées.

Selon un mode de réalisation particulier, ledit container dans lequel est déposé ledit insert pour culture cellulaire est un puits d'une plaque de culture cellulaire de 6, 8, 12, 24, ou 48 puits.

Parmi ces plaques de cultures on peut citer celles fournies en particulier par la société NUNC, BD FALCON, ou sous les références Millicell^{®} ou Costar^{®}.

De préférence, le fond de l'insert pour culture cellulaire est situé à une distance comprise entre 1mm et 2,5 mm du fond du container contenant ledit insert, notamment du fond du puits de la plaque de culture (ou du fond de la boite de Pétri selon l'appellation).

Un avantage du modèle de peau inflammée de l'invention est de pouvoir réaliser le criblage de composés anti-inflammatoires à grande échelle, notamment en utilisant à plusieurs reprises des échantillons (i.e. biopsies) de peau saine provenant du même donneur ou de donneurs distincts. Une pluralité de composés candidats peut être criblée, de plusieurs dizaines à plusieurs centaines, voire plusieurs milliers. Un intérêt d'obtenir des modèles de peau inflammée à partir de plusieurs donneurs est de pouvoir comparer la modulation interindividuelle du taux des marqueurs de l'inflammation et identifier ainsi les composés candidats les plus actifs sur la diminution de l'inflammation de la peau.

Encore un autre objet de l'invention concerne l'utilisation du modèle *ex vivo* de peau inflammée susceptible d'être obtenu par le procédé décrit précédemment pour l'identification des composés présentant un effet anti-inflammatoire.

La structure du modèle *ex vivo* de peau inflammée susceptible d'être obtenu par le procédé décrit précédemment permet une diffusion correcte des composés présentant un effet anti-inflammatoire après injection en sous-cutanée ou application topique, influençant *de facto* leur biodisponibilité.

Les composés identifiés selon cette utilisation de l'invention peuvent servir de base à l'élaboration de traitements thérapeutiques pour un patient souffrant d'inflammation cutanée aigüe ou chronique. Une telle utilisation selon l'invention permet d'envisager une thérapie personnalisable d'un patient à l'autre en fonction de l'intensité de l'effet anti-inflammatoires des différents composés testés sur les modèles de peau inflammée obtenus par le procédé de l'invention.

Parmi les pathologies inflammatoires de la peau on citera, mais sans s'y limiter, la dermatite atopique, le psoriasis, l'eczéma, dermatoses, dermites allergiques de contact, lichen, prurit, le syndrome de Netherton, l'ichtyose vulgaire...

Selon un mode de réalisation particulier de l'invention, le modèle *ex vivo* de peau inflammée susceptible d'être obtenu par le procédé décrit précédemment permet l'identification des composés destinés à traiter le psoriasis.

Un autre objet de l'invention concerne un kit de mise en œuvre du procédé de l'invention, ledit kit comprenant une biopsie de peau saine préalablement prélevée chez un mammifère, une composition A comprenant une quantité efficace d'un anticorps anti-CD3 et d'un anticorps anti-CD28, et optionnellement d'IL-2, d'une composition B comprenant au moins un mélange d'IL-1β, d'IL-23 et de TGFβ, et optionnellement un dispositif d'injection, en particulier par perfusion.

De préférence, la quantité efficace d'anti-CD3 dans la composition A varie de 10 ng/µl à 100 ng/µl, de préférence de 20 ng/µl à 80 ng/µl, et de manière particulièrement préférée de 30 ng/µl à 70 ng/µl.

De préférence, la quantité efficace d'anti-CD28 dans la composition A varie de 10 ng/µl à 100 ng/µl, de préférence de 20 ng/µl à 80 ng/µl, et de manière particulièrement préférée de 30 ng/µl à 70 ng/µl.

De préférence, la quantité efficace d'IL-2 dans la composition A varie de 1 ng/ml à 20 ng/ml, de préférence de 2 ng/ml à 15 ng/ml, et de manière particulièrement préférée de 4 ng/ml à 12 ng/ml.

Selon un mode de réalisation particulier, les quantités efficaces d'anticorps anti-CD3, d'anticorps anti-CD28 et d'IL-2 dans la composition A sont respectivement de 50 ng/µl, 50 ng/µl et 10 ng/ml.

De préférence, la concentration en IL-1β dans le mélange de la composition B varie de 1 ng/ml à 50 ng/ml, de préférence de 5 ng/ml à 30 ng/ml, et de manière particulièrement préférée de 7 ng/ml à 15 ng/ml.

De préférence, la concentration en IL-23 dans le mélange de la composition B varie de 10 ng/ml à 100 ng/ml, de préférence de 20 ng/ml à 80 ng/ml, et de manière particulièrement préférée de 30 ng/ml à 60 ng/ml.

De préférence, la concentration en TGF-β dans le mélange de la composition B varie de 1 ng/ml à 50 ng/ml, de préférence de 5 ng/ml à 30 ng/ml, et de manière particulièrement préférée de 7 ng/ml à 15 ng/ml.

Selon un mode de réalisation particulier, les quantités d'IL-1β, d'IL-23 et de TGF-β dans le mélange de la composition B sont respectivement de 10 ng/ml, 50 ng/ml et 10 ng/ml.

Dans un kit selon l'invention, la biopsie de peau saine est issue d'un fragment de peau qui ne présente aucun signe d'inflammation perceptible à l'œil (i.e. brèche cutanée, rougeur, gonflement, chaleur ou desquamation excessive, ...) ou n'exprime aucun marqueur de l'inflammation. En outre, ladite biopsie de peau saine comprend au moins l'épiderme, le derme et les annexes épidermiques, et optionnellement une partie du tissu sous-cutané encore appelé hypoderme.

De préférence, ladite biopsie de peau est prélevée chez un animal, en particulier un mammifère, et de préférence chez l'humain. Comme biopsies utilisables dans le kit de l'invention, on peut citer celles issues de déchets chirurgicaux ou d'abattoirs. Par « déchets chirurgicaux », on désigne des explants de peaux obtenus par un acte de chirurgie esthétique, notamment après blépharoplastie, rhinoplastie, abdominoplastie ou réduction mammaire. Les techniques de prélèvement de biopsie de peau sont bien connues de l'Homme du métier.

Dans un kit selon l'invention, le dispositif d'injection manuelle comprend une seringue sans volume mort dans l'aiguille de format 26S ou 22S.

Dans un kit selon l'invention, le dispositif d'injection par perfusion comprend une pompe osmotique reliée à un cathéter raccordé à une aiguille de format 28G ou 30G.

Encore un autre objet de l'invention concerne un kit comprenant :
- au moins un insert pour culture cellulaire (10), apte à être contenu dans un container ou un puits de boite de culture, et dont le fond est constitué d'une membrane poreuse (40), ledit insert contenant la biopsie de peau saine injectée obtenue à l'issue de l'étape a) du procédé de l'invention (30) enserrée dans une matrice solidifiée (20), ladite matrice étant en contact avec le bord interne de l'insert et la membrane poreuse, l'épiderme de la biopsie de peau étant en contact avec l'atmosphère et le derme et les annexes épidermiques de la biopsie de peau étant immergés dans la matrice solidifiée,
- d'une composition B comprenant au moins un mélange dIL-1β, d'IL-23 et de TGF-β.

Selon un autre mode de réalisation particulier, ledit kit de l'invention comprend :
- au moins un insert pour culture cellulaire (10), apte à être contenu dans un container ou un puits de boite de culture, et dont le fond est constitué d'une membrane poreuse (40), ledit insert contenant la biopsie de peau saine injectée obtenue à l'issue de l'étape a) du procédé de l'invention (30) enserrée dans une matrice solidifiée (20) contenant au moins le mélange d'IL-1β, d'IL-23 et de TGF-β, ladite matrice étant en contact avec le bord interne de l'insert et la membrane poreuse, l'épiderme de la biopsie de peau étant en contact avec l'atmosphère et le derme et les annexes épidermiques de la biopsie de peau étant immergés dans la matrice solidifiée,
- d'une composition B comprenant au moins un mélange d'IL-1β, d'IL-23 et de TGF-β.

Les exemples suivants sont donnés uniquement à titre d'illustration de l'objet de la présente invention dont ils ne constituent en aucune manière une limitation.

### EXEMPLES

### 1 - Préparation des biopsies de peau

Les biopsies de peau sont préparées à partir d'un prélèvement complet de peau, comprenant l'épiderme, le derme et l'hypoderme. A partir de ces biopsies, deux modes de réalisation sont envisageables en fonction de la nature des composés anti-inflammatoires à tester.

Dans un premier mode de réalisation, le tissu adipeux (i.e. l'hypoderme) est découpé à l'aide de ciseaux courbes afin de le séparer du derme. Les biopsies (épiderme et derme), dont l'épaisseur est de l'ordre de 3 mm, sont ensuite découpées à l'aide d'un emporte-pièce métallique. Ce premier mode de réalisation est destiné à tester des composés anti-inflammatoires par application topique.

Dans un second mode de réalisation, l'hypoderme est conservé. Les biopsies (épiderme, derme et hypoderme), dont l'épaisseur est de l'ordre de 1 cm, sont ensuite découpées à l'aide d'un emporte-pièce métallique. Ce second mode de réalisation est destiné à tester des composés anti-inflammatoires par injection en sous-cutané.

Une fois préparées, les biopsies sont maintenues en flottaison dans une solution saline tamponnée jusqu'à l'étape d'activation des cellules T résidentes.

### 2 - Activation des cellules T résidentes

A l'aide d'une pince, la biopsie de peau est maintenue à plat, derme sur le dessus. Une aiguille sans volume mort est introduite dans la biopsie par le côté du derme à l'aide d'une seringue de type Hamilton 701N ou 705N (pour les biopsies de 8 mm ou 15 mm de diamètre, respectivement). 10µl ou 35 µL de la solution d'activation (50 ng/µL anticorps anti-CD3, 50 ng/µL anticorps anti-CD28 et 10 ng/mL IL-2), pour les biopsies de 8 ou 15 mm de diamètre respectivement, sont injectés par le côté de la biopsie pour ne pas endommager l'épiderme.

Lorsque l'injection est réalisée par perfusion, 200 µL de la solution d'activation sont chargés dans la pompe osmotique (pour le modèle de pompe 2001D testé). La concentration de la solution a été ajustée selon la taille des biopsies (2,5 ng/µL anticorps anti-CD3, 2,5 ng/µL anticorps anti-CD28, 0,5 ng/mL IL-2 pour les biopsies de 8 mm de diamètre ; 8,75 ng/µL anticorps anti-CD3, 8,75 ng/µL anticorps anti-CD28, 1,75 ng/mL IL-2 pour les biopsies de 15 mm de diamètre.

La pompe est ensuite connectée à un cathéter à l'aide d'un modérateur de flux. La pompe est ensuite amorcée : elle est placée dans une solution saline tamponnée et incubée à 37°C pendant 3 heures.

A l'issue de cette incubation, l'extrémité du cathéter libre (non reliée à la pompe) est connectée à une aiguille. L'implantation de l'aiguille dans le derme de la biopsie est réalisée manuellement de la même façon que celle décrite précédemment : la peau est maintenue à plat à l'aide d'une pince, derme sur le dessus. L'aiguille est insérée dans le derme par le côté de la biopsie pour ne pas endommager l'épiderme.

Les biopsies implantées sont ensuite déposées dans un support de culture en flottaison sur du milieu de culture approprié. Les pompes sont maintenues immergées dans une solution saline tamponnée. L'ensemble est incubé pendant 24 heures dans un incubateur à 37°C, 5 % de CO2 et au taux maximal d'humidité.

A l'issue des 24 heures de perfusion, l'aiguille est retirée de la biopsie et la pompe ainsi déconnectée.

### 3 - Culture ex vivo

La biopsie de peau dont les cellules T résidentes ont été activées est montée sur une matrice liquide coulée dans un insert de culture selon le même procédé que celui utilisé pour le modèle NativeSkin^{®}, basé sur l'utilisation d'une matrice naturelle servant de support physique et de couche nourricière à des explants de peau ou d'épiderme humains cultivés *in vitro.* L'explant est ensuite cultivé en plaque (culture en incubateur, renouvellement quotidien du milieu de culture).

### 4 - Différenciation des cellules T en Th1/Th17

Dans le milieu de culture synthétique WILLIAM'S E, les compléments suivants sont ajoutés : 10 ng/mL IL-1β, 50 ng/mL IL-23 et 10 ng/mL TGFbeta. La culture est réalisée pendant 7 jours dans un incubateur à CO₂ à 37°C. Le milieu de culture contenant les compléments est changé quotidiennement.

A l'issue des étapes d'activation et de différentiation des cellules T résidentes du derme, les biopsies de peau portent les noms commerciaux de InflammaSkin^{®} lorsque qu'elles comprennent l'épiderme et le derme, ou de Hypo-InflammaSkin^{®} lorsque qu'elles comprennent l'épiderme, le derme et l'hypoderme.

### 5 - Analyse par histologie

### 5.1 - Coupe sur microtome

L'analyse peut se faire sur des échantillons inclus en bloc de paraffine par coupes sériées au microtome.

La biopsie de peau traitée selon les étapes a) et b) du procédé de l'invention est déshydratée par un bain d'alcool puis passage dans un bain de xylène. Un premier bain dans la paraffine permet de remplacer l'eau préalablement contenue dans l'explant de peau par la paraffine.

Les échantillons imprégnés de paraffine sont sortis de leur bain et transférés dans un récipient dont le fond est tapissé de papier absorbant, afin d'être apportés à proximité de la station d'inclusion.

Les échantillons, enfermés dans des cassettes d'histologie, sont plongés dans la paraffine liquide à 56°C pour faire refondre la paraffine qui les imprègne.

Pour chaque échantillon : la cassette d'histologie est ouverte, l'échantillon est éventuellement coupé en deux. Un moule d'inclusion est rempli de paraffine liquide, et l'échantillon (ou les 2 morceaux d'échantillon) est placé dans le moule et orienté dans le sens désiré pour la coupe. Le moule est en même temps transféré sur un support réfrigéré afin de solidifier la paraffine du fond du moule et d'y maintenir l'échantillon. Le couvercle de la cassette d'histologie sur lequel figure la référence de l'échantillon est placé par-dessus, de telle façon que la paraffine le traverse (possibilité d'ajouter de la paraffine si besoin), puis le tout est placé au froid (réfrigérateur, freezer, chambre froide...) plusieurs minutes (5-6), afin de solidifier la paraffine en bloc, emprisonnant l'échantillon dans la bonne orientation et le couvercle de la cassette d'histologie qui deviendra le support du bloc.

Une fois le bloc bien solide, celui-ci est démoulé. L'excès de paraffine est éventuellement gratté à l'aide d'une spatule sur les côtés du couvercle de la cassette d'inclusion.

Des coupes sériées d'épaisseur variant de 4 à 5 µm sont alors réalisées sur toute la longueur du bloc de paraffine contenant l'échantillon.

La figure 2 illustre les résultats d'une telle coloration réalisée sur biopsie de peau traitée selon le procédé de l'invention après 5 ou 7 jour de culture dans le milieu de polarisation des cellules T résidentes activées en cellules Th1 et/ou Th17. L'analyse du modèle par histologie (ex : coloration Hématoxyline et Eosine) permet d'attester de la l'apparition de spongiose (S) au niveau de l'épiderme ainsi que de la formation d'élongation des crêtes épidermales (E) après 7 jours de culture des biopsies activées en milieu de polarisation.

Des analyses ont été réalisées en histologie, à l'aide d'immunomarquages anti-CD207, anti-CD3, anti-HLA-DR et anti-tryptase afin d'identifier certaines sous-populations cellulaires contenues dans les biopsies de peau saine. Une contre-coloration nucléaire a été réalisée avec du DAPI. Les résultats de la figure 3 montrent bien que les biopsies de peau saine contiennent des cellules de Langerhans (CD207), des cellules T résidentes (CD3), des cellules dendritiques (HLA-DR) et des mastocytes (Tryptase) à T0 et après 7 jours de culture (T7).

### 5.2 - Cryosection

L'analyse peut également se faire sur coupe congelée afin de ne pas altérer la fluorescence des cellules / du(es) sphéroïde(s) à la déshydratation.

La culture de la biopsie de peau traitée selon les étapes a) et b) du procédé de l'invention est arrêtée en fixant l'échantillon 24h dans 10 fois son volume de formaline 10%. L'explant est ensuite rincé dans 10 fois son volume de PBS, épongé sur un papier absorbant et transféré dans un tube EPPENDORF. Le tube EPPENDORF est plongé dans l'azote liquide jusqu'à la congélation de l'explant.

Dans l'enceinte du cryostat, l'explant congelé est inclus en OCT puis des tranches d'épaisseur constante de 3 à 15 µm de la partie de l'explant contenant le/le(s) sphéroïde(s) implanté(s) sont réalisées. Les tranches sont déposées sur lames qui sont séchées pendant quelques heures au minimum.

Une coloration classique à l'Hématoxyline et Eosine est réalisée.

### 6- Validation du modèle de peau inflammée

### 6.1 - Marqueurs de l'inflammation

Les cytokines IL-17A, IL-22 et IFNγ produites par le modèle ont été analysées dans les milieux de culture par dosage ELISA à l'aide d'un kit de détection spécifique provenant de chez Meso Scale Discovery (U-PLEX TH17 Combo 2 Human Référence K15076K) et à l'aide d'une plateforme de dosage multiplex (MESO QuickPlex SQ 120).

La figure 4 illustre les résultats obtenus lors du dosage des cytokines sur différents modèles. Les modèles NativeSkin^{®} sont des biopsies de peau comprenant l'épiderme et le derme, et sont dépourvues d'hypoderme. A l'inverse des modèles NativeSkin^{®} standard (1) ou bien NativeSkin^{®} cultivés en présence du cocktail de polarisation pro Th17/Th1 (2), les cytokines IL-17A (figure 4A) et IL-22 (figure 4B) sont produites uniquement par le modèle InflammaSkin^{®} (3) obtenu par le procédé de l'invention.

### 6.2 - Activation des kératinocytes

A l'issue de l'étape b) du procédé de l'invention, les modèles *ex vivo* de peau inflammée présentent des signes caractéristiques comparables à ceux observés dans les lésions psoriasiques de patients atteints de psoriasis. Le tableau ci-dessous illustre l'apparition de certains de ces marqueurs.

| | **Biopsie contrôle** | | | **Biopsie traitée selon les étapes a) et b) du procédé de l'invention** | | |
|---|---|---|---|---|---|---|
| | **T0** | **T5** | **T7** | **T0** | **T5** | **T7** |
| **Apoptose** | - | - | - | - | + | + |
| **S100A7** | - | - | - | - | + | ++ |
| **KRT16** | - | - | +/- | - | + | +++ |

Dès 5 jours d'incubation de la biopsie de peau en présence du mélange IL-1β, IL-23 et TGF-β, on observe l'apparition des marqueurs S100A7 (psoriasine) et la kératine 16 (KRT16), caractéristiques d'une hyperprolifération des kératinocytes. Les taux de ces marqueurs augmentent encore 7 jours après le début la polarisation des cellules T CD4⁺ activées en Th1/Th17.

En outre, on observe l'apparition d'apoptose au sein des kératinocytes de l'épiderme du modèle ex vivo de peau inflammée obtenu par le procédé de l'invention.

### 7- Criblage de composés anti-inflammatoires

Afin de tester la pertinence du modèle de peau inflammée obtenu par le procédé de l'invention, des composés connus pour leur effet anti-inflammatoires ont été testés. Il s'agit de la betamethasone di-propionate et de l'inhibiteur de PDE4. Des applications topiques de ces produits (volume constant de 10 µL sur des biopsies de 8 mm de diamètre) ont été réalisées immédiatement après activation et production du modèle InflammaSkin^{®} (traitement prophylactique) ou après 3 jours de culture sans traitement (traitement thérapeutique), et répétées quotidiennement jusqu'au 7^{e} jour de culture.

L'analyse par histologie (figure 5) montre que l'intégrité et la structure de la peau et plus particulièrement de l'épiderme, et la viabilité des cellules de l'épiderme sont préservées lors du traitement avec le betamethasone di-propionate et l'inhibiteur de PDE4 en traitement prophylactique comparativement à leurs contrôles placebo respectifs.

L'analyse de la sécrétion de cytokines pro-inflammatoires IL-17A), IL-22, IFNγ et TNFα (figures 6 à 9) montre que :
- la sécrétion d'IL-17A (figure 6) est fortement inhibée par la betamethasone di-propionate (3) et l'inhibiteur de PDE4 (5) en traitement prophylactique comparativement à leurs contrôles placébo respectifs (figure 6A), alors que seule la betamethasone di-propionate (3) inhibe la sécrétion d'IL-17A en traitement thérapeutique (figure 6B),
- la sécrétion d'IL-22 (figure 7) est fortement inhibée par la betamethasone di-propionate (3) et encore plus fortement par l'inhibiteur de PDE4 (5) en traitement prophylactique comparativement à leurs contrôles placébo respectifs (figure 7A), alors que seule la betamethasone di-propionate (3) inhibe la sécrétion d'IL-22 en traitement thérapeutique (figure 7B),
- la sécrétion d'IFNy (figure 8) est fortement inhibée par la betamethasone di-propionate (3) et l'inhibiteur de PDE4 (5) en traitement prophylactique comparativement à leurs contrôles placébo respectifs (figure 8A), alors que seule la betamethasone di-propionate (3) inhibe la sécrétion d'IFNy en traitement thérapeutique (figure 8B),
- la sécrétion de TNFα (figure 9) est fortement inhibée par la betamethasone di-propionate (3) et l'inhibiteur de PDE4 (5) non seulement en traitement prophylactique comparativement à leurs contrôles placébo respectifs (figure 9A), mais également en traitement thérapeutique (figure 9B).

### 8- Incorporation de l'anticorps anti-CD3 dans les cellules du derme

Un test comparatif relatif à l'incorporation d'un anticorps anti-CD3 a été réalisé sur des biopsies de peau humaine. 40 ng/µl d'anticorps anti-CD3e couplé au fluorochrome Alexa Fluor 647 (ThermoFisher, ref. A51001) ont été soit injectés directement dans le derme d'une biopsie de peau humaine de 4 mm d'épaisseur et de 8 mm de diamètre, soit ajouté au milieu de culture dans lequel la biopsie de peau est maintenue en flottaison. Après 24 heures de culture, les biopsies de peau sont fixées dans une solution de formaline à 10% et incluses dans des blocs de paraffine. Des coupes sériées d'épaisseur constante de 5 µm des biopsies sont réalisées. Les noyaux des cellules sont marqués au DAPI. Un microscope à fluorescence Leica DM5000 est utilisé pour l'analyse.

L'incorporation de l'anticorps anti-CD3e dans les cellules du derme est matérialisée par des flèches dans deux biopsies distinctes (réplicats 1 et 2) ayant subies une injection de cet anticorps directement dans le derme (figure 11, panneaux de gauche - II). A fort grossissement (figure 12), il est possible de voir que l'incorporation de l'anticorps anti-CD3e s'effectue dans le cytoplasme de quelques unes des cellules dermales.

A *contrario,* pour une seule des deux biopsies ayant été incubées dans un milieu de culture comprenant l'anticorps anti-CD3e, la détection de cet anticorps n'est visible, et matérialisée par une étoile, qu'à la surface de l'épiderme (figure 11 panneau de droite - AMC/R1). Aucune cellule du derme n'a pu incorporer l'anticorps anti-CD3e par diffusion passive.

Ces résultats montrent bien que l'anticorps anti-CD3 atteint les cellules du derme uniquement s'il est injecté directement dans ce tissu.

Des résultats similaires peuvent être obtenus avec l'anticorps anti-CD28.

### 9- Criblage de composés anti-inflammatoires

Afin de tester la pertinence du modèle de peau inflammée obtenu par le procédé de l'invention (Hypo-InflammaSkin^{®}), un anticorps connu pour son effet anti-inflammatoire a été testé. Il s'agit de l'anticorps anti-TNFα adalimumab. Des biopsies de peau inflammée (Hypo-InflammaSkin^{®} - I) d'une épaisseur de 1 cm ont été obtenues par le procédé de l'invention. Une injection sous-cutanée (i.e. dans le derme) de 50 nM d'adalimumab a été réalisée à T0 (traitement prophylactique, Hypo-InflammaSkin^{®} - I + anti-TNFα - P) ou après 3 jours de culture *ex vivo* de la biopsie inflammée (traitement thérapeutique, Hypo-InflammaSkin^{®} - I + anti-TNFα - T). Après 7 jours de culture *ex vivo,* les milieux de culture sont prélevés et les biopsies de peau sont fixées dans une solution de formaline à 10% puis incluses dans des blocs de paraffine. Des coupes sériées d'épaisseur constante de 5 µm des biopsies sont réalisées. L'aspect morphologique des biopsies de peau est étudié en microscopie optique grâce à une coloration Hématoxyline et Eosine. La présence de psoriasine (S110A7) est mise en évidence par microscopie à fluorescence (microscope Leica DM 5000) en utilisant un anticorps anti-S100A7 couplé à un fluorochrome. Les noyaux des cellules sont marqués au DAPI. Des biopsies de peau non-inflammée (HypoSkin^{®} - biopsies comprenant l'épiderme, le derme et l'hypoderme) servent de contrôle.

L'analyse par histologie (figure 13) montre que l'intégrité et la structure de la peau et plus particulièrement de l'épiderme, et la viabilité des cellules de l'épiderme sont préservées lors du traitement avec l'anticorps anti-TNFα adalimumab en traitement prophylactique (Hypo-InflammaSkin^{®} - I + anti-TNFα - P) et en traitement thérapeutique (Hypo-InflammaSkin^{®} - I + anti-TNFα - T) comparativement à une biopsie de peau inflammée non traitée par cet anticorps (Hypo-InflammaSkin^{®} - I).

De plus, la psoriasine devient quasi indétectable dans les échantillons de biopsie inflammée et traitée par l'anti-TNF-α, attestant ainsi de la résolution de l'inflammation.

L'analyse de la sécrétion de la cytokine pro-inflammatoire IL-22 montre que la sécrétion d'IL-22 (figure 14) est fortement inhibée par l'anticorps anti-TNFα en traitement prophylactique (Hypo-InflammaSkin^{®} - I + anti-TNFα - P) et en traitement thérapeutique (Hypo-InflammaSkin^{®} - I + anti-TNFα - T) comparativement à une biopsie de peau inflammée non-traitée par cet anticorps Hypo-InflammaSkin^{®} - I).

Ces résultats montrent que le modèle *ex vivo* de peau inflammée obtenu par le procédé de l'invention permet le criblage de composés anti-inflammatoires, et en particulier anti-psoriasiques, dont la voie d'administration est une injection sous-cutanée.

## Revendications

1. Un procédé *in vitro* pour obtenir un modèle *ex vivo* de peau inflammée comprenant les étapes suivantes :
a) injecter, dans le derme d'une biopsie de peau saine préalablement prélevée chez un mammifère, une composition comprenant une quantité efficace pour l'activation des cellules T résidentes du derme d'un anticorps anti-CD3 et d'un anticorps anti-CD28,
b) incuber la biopsie de peau injectée obtenue à l'étape a) en présence d'une composition comprenant une quantité efficace, pour obtenir la polarisation des cellules T activées à l'étape a) en LTh1 et/ou LTh17 et la synthèse de marqueurs de l'inflammation, d'au moins un mélange d'IL-1β, d'IL-23 et de TGF-β.

2. Le procédé selon la revendication 1, dans lequel la composition injectée à l'étape a) du procédé comprend également une quantité efficace d'IL-2.

3. Le procédé selon la revendication 1 ou 2, dans lequel l'étape a) est réalisée manuellement.

4. Le procédé selon l'une quelconque des revendications 1 à 3, dans lequel l'étape b) d'incubation dure à minima 5 jours.

5. Le procédé selon l'une quelconque des revendications 1 à 4, lequel comprend entre les étapes a) et b) une étape supplémentaire a') de dépôt de la biopsie de peau injectée obtenue à l'issue de l'étape a) sur une matrice liquide apte à se solidifier, ladite matrice étant elle-même contenue dans un insert pour culture cellulaire dont le fond est constitué d'une membrane poreuse et ledit insert étant disposé dans un container ou puits, de sorte à permettre, une fois la matrice solidifiée, le maintien de l'intégrité 3D de la biopsie de peau.

6. Le procédé selon l'une quelconque des revendications 1 à 5, dans lequel les concentrations dans la composition injectée à l'étape a) du procédé sont comprises entre :
- entre 10 ng/µl et 100 ng/µl, de préférence entre 20 ng/µl et 80 ng/µl, et de manière particulièrement préférée de 30 ng/µl à 70 ng/µl pour les anticorps anti-CD3 et anti-CD28, et optionnellement,
- entre 1 ng/ml à 20 ng/ml, de préférence de 2 ng/ml à 15 ng/ml, et de manière particulièrement préférée de 4 ng/ml à 12 ng/ml pour l'IL-2.

7. Le procédé selon l'une quelconque des revendications 1 à 6, dans lequel les concentrations dans le au moins un mélange de la composition utilisée à l'étape b) du procédé sont comprises entre :
- entre 1 ng/ml et 50 ng/ml, de préférence entre 5 ng/ml et 30 ng/ml, et de manière particulièrement préférée de 7 ng/ml à 15 ng/ml pour l'IL-1β et le TGFβ, et
- entre 10 ng/ml à 100 ng/ml, de préférence de 20 ng/ml à 80 ng/ml, et de manière particulièrement préférée de 30 ng/ml à 60 ng/ml pour l'IL-23.

8. Procédé selon l'une quelconque des revendications 1 à 7, lequel vise en outre à évaluer l'efficacité anti-inflammatoire d'un composé et comprend les étapes supplémentaires suivantes :
c) mettre en contact, avec un composé candidat, le modèle de peau inflammée obtenu à l'issue de l'étape b) et comprenant des cellules T polarisées en LTh1 et/ou LTh17 ;
d) mesurer le niveau d'expression d'au moins deux marqueurs de l'inflammation dans le modèle de peau inflammée;
e) comparer le niveau d'expression desdits au moins deux marqueurs de l'inflammation obtenus à l'étape d) avec leur niveau d'expression contrôle ;
f) identifier l'efficacité anti-inflammatoire dudit composé candidat lorsque le niveau d'expression desdits au moins deux marqueurs de l'inflammation mesuré à l'étape d) est inférieur à leur niveau d'expression contrôle.

9. Procédé selon la revendications 8, lequel comprend également à l'étape d) la mesure du niveau d'expression d'au moins trois, de préférence d'au moins cinq marqueurs de l'inflammation choisis parmi IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 et CCL20.

10. Modèle *ex vivo* de peau inflammée comprenant un derme, un épiderme, des annexes épidermiques, un hypoderme et des cellules Th1 et/ou Th17, lequel est susceptible d'être obtenu par un procédé selon l'une quelconque des revendications 1 à 7 et est **caractérisé par** l'expression au niveau protéique d'au moins deux marqueurs de l'inflammation, de préférence d'au moins cinq marqueurs de l'inflammation choisis parmi IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 et CCL20.

11. Insert pour culture cellulaire (10), apte à être contenu dans un container ou un puits de boite de culture, et dont le fond est constitué d'une membrane poreuse (40), ledit insert contenant le modèle *ex vivo* de peau inflammée (30) selon la revendication 10 enserré dans une matrice solidifiée (20), ladite matrice étant en contact avec le bord interne de l'insert et la membrane poreuse, l'épiderme du modèle *ex vivo* de peau inflammée étant en contact avec l'atmosphère et le derme et les annexes épidermiques du modèle *ex vivo* de peau inflammée étant immergés dans la matrice solidifiée.

12. Utilisation du modèle *ex vivo* de peau inflammée selon la revendication 10 ou de l'insert pour culture cellulaire selon la revendication 11 pour identifier des composés anti-inflammatoires, en particulier anti-psoriasiques.

13. Kit pour la mise en œuvre du procédé selon l'une quelconque des revendications 1 à 9 comprenant une biopsie de peau saine préalablement prélevée chez un mammifère, une composition A comprenant une quantité efficace d'un anticorps anti-CD3 et d'un anticorps anti-CD28, et optionnellement d'IL-2, d'une composition B comprenant au moins un mélange d'IL-1β, d'IL-23 et de TGF-β.

14. Kit selon la revendication 12, lequel comprend en outre un dispositif d'injection.

15. Procédé selon la revendication 1 à 7, dans lequel la biopsie de peau saine utilisée à l'étape a) comprend un derme, un épiderme, des annexes épidermiques, et optionnellement un hypoderme.

## Patentansprüche

1. *In-vitro*-Verfahren zur Erzeugung eines *ex-vivo*-Modells entzündeter Haut, die folgenden Schritte umfassend:
a) Injektion in die Dermis einer Gewebeprobe gesunder Haut, die vorab bei einem Säugetier genommen wurde, eines Gemisches, das eine zur Aktivierung der residenten T-Zellen der Dermis wirksame Menge eines Antikörpers anti-CD3 und eines Antikörpers anti-CD28 umfasst,
b) Inkubation der in Schritt a) erhaltenen injizierten Gewebeprobe in Anwesenheit eines Gemisches, das eine zur LTh1- und/oder LTh17-Polarisierung der in Schritt a) aktivierten T-Zellen und zur Synthese von Entzündungs-Markern, mindestens eines Gemisches von IL-1β, IL-23 und TGF-β, wirksame Menge enthält.

2. Verfahren nach Patentanspruch 1, in dem das in Schritt a) des Verfahrens injizierte Gemisch außerdem eine wirksame Menge an IL-2 enthält.

3. Verfahren nach Patentanspruch 1 oder 2, in dem der Schritt a) manuell ausgeführt wird.

4. Verfahren nach irgendeinem der Patentansprüche 1 bis 3, in dem der Inkubationsschritt b) mindestens 5 Tage dauert.

5. Verfahren nach irgendeinem der Patentansprüche 1 bis 4, zwischen den Schritten a) und b) einen zusätzlichen Schritt a') umfassend, die injizierte Hautgewebeprobe, die in Schritt a) erhalten wurde, auf eine flüssige Matrix zu legen, die in der Lage ist, sich zu verfestigen, wobei die genannte Matrix selbst sich in einem Zellkultureinsatz befindet, dessen Boden aus einer porösen Membran besteht, und der genannte Einsatz in einem Behälter oder Well angeordnet ist, um nach Verfestigung der Matrix die Aufrechterhaltung des 3D-Zusammenhaltes der Hautgewebeprobe zu ermöglichen.

6. Verfahren nach irgendeinem der Patentansprüche 1 bis 5, in dem die Konzentrationen im in Schritt a) des Verfahrens injizierten Gemisch
- für die Antikörper anti-CD3 und anti-CD28 zwischen 10 ng/µl und 100 ng/µl liegen, vorzugsweise zwischen 20 ng/µl und 80 ng/µl, und besonders bevorzugt zwischen 30 ng/µl und 70 ng/µl, und optional
- für IL-2 zwischen 1 ng/µl und 20 ng/µl liegen, vorzugsweise zwischen 2 ng/µl und 15 ng/µl, und besonders bevorzugt zwischen 4 ng/µl und 12 ng/µl.

7. Verfahren nach irgendeinem der Patentansprüche 1 bis 6, in dem die Konzentrationen in der mindestens einen Mischung des in Schritt b) des Verfahrens verwendeten Gemisches
- für IL-1β und TGFß zwischen 1 ng/ml und 50 ng/ml liegen, vorzugsweise zwischen 5 ng/ml und 30 ng/ml, und besonders bevorzugt zwischen 7 ng/ml und 15 ng/ml, und
- für IL-23 zwischen 10 ng/ml und 100 ng/ml liegen, vorzugsweise zwischen 20 ng/ml und 80 ng/ml, und besonders bevorzugt zwischen 30 ng/ml und 60 ng/ml.

8. Verfahren nach irgendeinem der Patentansprüche 1 bis 7, das außerdem darauf abzielt, die entzündungshemmende Wirksamkeit einer Zubereitung zu bewerten, und die folgenden zusätzlichen Schritte umfasst:
c) Kontaktieren des im Schritt b) erzeugten und LTh1- und/oder LTh17-polarisierte T-Zellen enthaltenden Modells entzündeter Haut mit einem Zubereitungskandidaten,
d) Messung des Expressionsniveaus mindestens zweier Entzündungsmarker im Modell entzündeter Haut,
e) Vergleich der Expressionsniveaus der genannten, in Schritt d) erhaltenen mindestens zwei Entzündungsmarker mit ihrem Kontrollexpressionsniveau,
f) Identifikation der Wirksamkeit zur Entzündungshemmung des genannten Zubereitungskandidaten, wenn das Expressionsniveau der genannten, in Schritt d) gemessenen mindestens zwei Entzündungsmarker niedriger ist, als ihr Kontrollexpressionsniveau.

9. Verfahren nach Patentanspruch 8, außerdem in Schritt d) die Messung des Expressionsniveaus von mindestens drei, vorzugsweise von mindestens fünf Entzündungsmarkern, ausgewählt unter IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 und CCL20.

10. Ex-vivo-Modell entzündeter Haut, eine Dermis, eine Epidermis, Epidermisanhänge, eine Hypodermis und Th1- und/oder Th17-Zellen umfassend, das nach dem Verfahren nach irgendeinem der Patentansprüche 1 bis 7 hergestellt worden sein kann und das durch Proteinexpression mindestens zweier Entzündungsmarker, vorzugsweise von mindestens fünf Entzündungsmarkern, ausgewählt unter IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18 und CCL20, gekennzeichnet ist.

11. Zellkultur-Einsatz (10), geeignet, in einem Behälter oder Well einer Kulturschale angeordnet zu werden, und dessen Boden aus einer porösen Membran (40) besteht, wobei der genannte Einsatz das *ex-vivo*-Modell entzündeter Haut (30) nach Patentanspruch 10 in einer verfestigten Matrix (20) eingeschlossen enthält, wobei die genannte Matrix mit dem Innenrand des Einsatzes und der porösen Membran in Berührung steht, wobei die Epidermis des *ex-vivo-*Modells entzündeter Haut mit der Atmosphäre in Kontakt steht, während die Dermis und die Epidermis-Anhänge des *ex-vivo-*Modells entzündeter Haut in die verfestigte Matrix eingebettet ist.

12. Gebrauch des *ex-vivo*-Modell entzündeter Haut nach Patentanspruch 10 oder des Zellkultureinsatzes nach Patentanspruch 11 zur Identifizierung entzündungshemmender, insbesondere Psoriasis-hemmender Zubereitungen.

13. Kit zur Ausführung des Verfahrens nach irgendeinem der Patentansprüche 1 bis 9, eine Gewebeprobe gesunder Haut, die vorab bei einem Säugetier abgenommen wurde, ein Gemisch A, das eine wirksame Menge eines Antikörpers anti-CD3 und eines Antikörpers anti-CD28 und optional von IL-2 enthält, ein Gemisch B, das mindestens eine Mischung von IL-1β, IL-23 und TGF-β umfasst, umfassend.

14. Kit nach Patentanspruch 12, das außerdem eine Injektionsvorrichtung umfasst.

15. Verfahren nach Patentanspruch 1 bis 7, in dem die Gewebeprobe gesunder Haut, die in Schritt a) verwendet wird, eine Dermis, eine Epidermis, Epidermisanhänge, und optional eine Hypodermis umfasst.

## Claims

1. An *in vitro* method for obtaining an *ex vivo* model of inflamed skin comprising the following steps:
a) injecting, into the dermis of a healthy skin biopsy previously taken from a mammal, a composition comprising an effective amount for activating dermal resident T cells of an anti-CD3 antibody and an anti-CD28 antibody,
b) incubating the injected skin biopsy obtained in step a) in the presence of a composition comprising an effective amount of at least one mixture of IL-1β, IL-23, and TGF-β for obtaining the polarization of the T cells activated in step a) into LTh1 and/or LTh17 and the synthesis of inflammation markers.

2. The method according to claim 1, wherein the composition injected at the step a) of the method further comprises an effective amount of IL-2.

3. The method according to claim 1 or 2, wherein the step a) is carried out manually.

4. The method according to any one of claims 1 to 3, wherein the incubation step b) lasts at least 5 days.

5. The method according to any one of claims 1 to 4, which comprises between steps a) and b) an additional step a') of depositing the injected skin biopsy obtained at the end of step a) on a liquid matrix capable of solidifying, said matrix being itself contained in a cell culture insert the bottom of which consists of a porous membrane and said insert being arranged in a container or well, so as to allow, once the matrix has solidified, the 3D integrity of the skin biopsy to be maintained.

6. The method according to any of claims 1 to 5, wherein the concentrations in the composition injected in step (a) of the method are the following ones:
- between 10 ng/µl and 100 ng/µl, preferably between 20 ng/µl and 80 ng/µl, and particularly preferably from 30 ng/µl to 70 ng/µl for the anti-CD3 and anti-CD28 antibodies, and optionally,
- between 1 ng/ml and 20 ng/ml, preferably from 2 ng/ml to 15 ng/ml, and particularly preferably from 4 ng/ml to 12 ng/ml for IL-2.

7. The method according to any of claims 1 to 6, wherein the concentrations in the at least one mixture of the composition used in step b) of the method are the following ones:
- between 1 ng/ml and 50 ng/ml, preferably between 5 ng/ml and 30 ng/ml, and particularly preferably from 7 ng/ml to 15 ng/ml for IL-1β and TGFβ, and
- between 10 ng/ml and 100 ng/ml, preferably from 20 ng/ml to 80 ng/ml, and particularly preferably from 30 ng/ml to 60 ng/ml for IL-23.

8. The method according to any one of claims 1 to 7, which further aims at evaluating the anti-inflammatory efficacy of a compound and comprises the following additional steps:
c) Contacting the model of inflamed skin obtained at the end of step b) comprising LTh1- and/or LTh17-polarized T cells with a candidate compound and;
d) measuring the expression level of at least two inflammation markers of the model of inflamed skin;
e) comparing the expression level of said at least two inflammation markers obtained in step d) with their control expression level;
f) identifying the anti-inflammatory efficacy of said candidate compound when the expression level of said at least two inflammation markers measured in step d) is lower than their control expression level.

9. The method according to claim 8, which also in step d) includes measuring the expression level of at least three, in particular at least four, preferably at least five, and particularly preferably, at least six inflammation markers selected from the group consisting of IL-8, IL-17A, IL-22, IL-23, IFNy, TNFα, S100A7, S100A8, S100A9, S100A12, SERPINB3, SERPINB4, SERPINB13, DEFB4, KRT6A, KRT16, KRT17, CXCL9, CXCL10, CCL18, and CCL20.

10. An *ex vivo* model of inflamed skin comprising a dermis, an epidermis, epidermal appendages, and Th1 and/or Th17 cells, which can be obtained by a method according to any one of claims 1 to 7 and is **characterized by** the expression of at least two inflammation markers, preferably at least five inflammation markers selected from the group consisting of IL-8, IL-17A, IL-22, IL-23, IFNγ, TNFα, S100A7, S100A8, S100A9, S100A12, SERPINB3, SERPINB4, SERPINB13, DEFB4, KRT6A, KRT16, KRT17, CXCL9, CXCL10, CCL18, and CCL20.

11. A cell culture insert (10), suitable for being contained in a container or a culture box well, and the bottom of which consists of a porous membrane (40), said insert containing the *ex vivo* model of inflamed skin (30) according to claim 10 embedded in a solidified matrix (20), said matrix being in contact with the inner edge of the insert and the porous membrane, the epidermis of the *ex vivo* model of inflamed skin being in contact with the atmosphere and the dermis and the epidermal appendages of the *ex vivo* model of inflamed skin being immersed in the solidified matrix.

12. A use of the *ex vivo* model of inflamed skin according to claim 10 or of the cell culture insert according to claim 11 for identifying anti-inflammatory compounds, in particular anti-psoriatic compounds.

13. A kit for implementing the method according to any of claims 1 to 9 comprising a healthy skin biopsy previously taken from a mammal, a composition A comprising an effective amount of an anti-CD3 antibody and an anti-CD28 antibody, and optionally IL-2, a composition B comprising at least a mixture of IL-1β, IL-23, and TGF-β,

14. The kit according to claim 13, which further comprises an injection device

15. The method according to the claim 1 to 7, wherein the healthy skin biopsy used at the step a) comprises a dermis, an epidermis, epidermic annexes and optionally a hypodermis.
